# EUROPEAN PATENT SPECIFICATION

(11) **EP 1 613 752 B1**
(45) Date of publication and mention of the grant of the patent: **23.05.2007**
(21) Application number: 03786456.8
(22) Date of filing: 04.12.2003
(51) Int. Cl.: C12N 15/82

(54) **CHIMAERIC PROTEIN CONTAINING CYSTEINE PROTEASE OF LIVER FLUKE FUSED TO HEPATITIS B CORE PROTEIN OR UBIQUITIN, PLANTS EXPRESSING SAID PROTEIN, AND USES THEREOF AS VACCINE**
CHIMAERES PROTEIN ENTHALTEND DIE CYSTEIN PROTEASE AUS DEM GROSSEN LEBEREGEL FUSIONIERT AN DAS HEPATITIS B CORE PROTEIN ODER UBIQUITIN; PFLANZEN, WELCHES DIESES PROTEIN EXPRIMIEREN, SOWIE DEREN VERWENDUNG ALS VAKZINE
PROTEINE CHIMERIQUE CONTENANT LA CYSTEINE PROTEASE DE LA DOUVE DU FOIE FUSIONNEE A LA PROTEINE DE NOYAU DU VIRUS DE L'HEPATITE B OU A L'UBIQUITINE; PLANTES EXPRIMANT LADITE PROTEINE; ET SON UTILISATION COMME VACCIN

(30) Priority: 04.12.2002 PL 35751702
(43) Date of publication of application: 11.01.2006
(73) Proprietor: Institute of Bioorganic Chemistry, PL-61-704 Poznan (PL); Institute of Biotechnology Antibiotics, PL-02-515 Warsaw (PL)
(72) Inventor: LEGOCKI, Andrzej, B., PL-61-614 Poznan (PL); MIEDZINSKA, Katarzyna, PL-63-900 Rawicz (PL); CZAPLINSKA, Magdalena, PL-62-051 Leczyca (PL); MODELSKA, Anna, PL-61-663 Poznan (PL); PNIEWSKI,Tomasz, PL-61-386 Poznan (PL); PLUCIENNICZAK, Andrzej, PL-04-360 Warszawa (PL); KEZIK, Malgorzata, PL-00-137 Warsawa (PL); POREBSKA, Anna, PL-02-615 Warszawa (PL); WEDRYCHOWICZ, Halina, PL-04-391 Warsawa (PL); MIESZCZANEK, Juliusz, PL-02-786 Warsawa (PL); JEDLINA-PANASIUK, Luiza, PL-00-818 Warsawa (PL); WOJCIECHOWICZ, Jacek, 61-616 Poznan (PL)
(74) Representative: Twardowska, Aleksandra
(86) International application number: PCT/PL2003/000135
(87) International publication number: WO 2004/050883

(56) References cited:
- WO-A-89/06658
- WO-A-94/09142
- WO-A-94/17820
- WO-A-94/28925
- WO-A-99/42472
- TARAR M R ET AL: "Expression of a human cytomegarovirus gp58 antigenic domain fused to the hepatitis B virus nucleocapsid protein" FEMS IMMUNOLOGY AND MEDICAL MICROBIOLOGY, ELSEVIER SCIENCE B.V., AMSTERDAM, NL, vol. 16, no. 3-4, 1996, pages 183-192, XP002973530 ISSN: 0928-8244
- SCHOEDEL F ET AL: "HEPATITIS B VIRUS CORE AND E ANTIGEN: IMMUNE RECOGNITION AND USE ASA VACCINE CARRIER MOIETY" INTERVIROLOGY, XX, XX, vol. 39, no. 1/2, 1996, pages 104-110, XP000891467 ISSN: 0300-5526
- SCHODEL F ET AL: "Hybrid hepatitis B virus core antigen as a vaccine carrier moiety: I. Presentation of foreign epitopes" JOURNAL OF BIOTECHNOLOGY, ELSEVIER SCIENCE PUBLISHERS, AMSTERDAM, NL, vol. 44, no. 1, 26 January 1996 (1996-01-26), pages 91-96, XP004036853 ISSN: 0168-1656
- WEDRYCHOWICZ H ET AL: "INVESTIGATION OF CYSTEINE PROTEINASE AS A POTENTIAL VACCINE CANDIDATE AGAINST FASCIOLA HEPATICA INFECTION" IMMUNOLOGY LETTERS, AMSTERDAM, NL, vol. 73, September 2000 (2000-09), page 272, XP000982901 ISSN: 0165-2478
- KOFTA W ET AL: "Successful DNA immunisation of rats against fasciolosis" VACCINE, BUTTERWORTH SCIENTIFIC. GUILDFORD, GB, vol. 18, no. 26, July 2000 (2000-07), pages 2985-2990, XP004199092 ISSN: 0264-410X cited in the application
- WEDRYCHOWICZ H ET AL: "The immune response of rats to vaccination with the cDNA or protein forms of the cysteine proteinase of Fasciola hepatica." VETERINARY IMMUNOLOGY AND IMMUNOPATHOLOGY, vol. 94, no. 1-2, 15 July 2003 (2003-07-15), pages 83-93, XP002291369 ISSN: 0165-2427

## Description

This invention relates to chimaeric proteins, sequence construct, plant cell, the method of manufacturing of the chimaeric protein and the transgenic plant, the transgenic plant, the application of transgenic plants in the manufacture of oral vaccines, a pharmaceutical composition, vaccine against the liver fluke, as well as a method for the induction of immunity against infections by the liver fluke in mammals. In general the invention relates to a method for inducing an immune response, encompassing the application of the vaccine through the digestive tract. The subjects of the invention serve to produce oral vaccines against the liver fluke.

The liver fluke (*Fasciola hepatica*) is an internal parasite of cattle. It is classified as a digenous fluke, meaning that in its life cycle there are several developmental stages. These take place with the aid of an intermediate host, the snail, *Lymnea truncatula.* The animal is attacked following the ingestion of plants, which are contaminated with the encysted invasive form, metacercaria. In the digestive tract of vertebrates, the cyst sac dissolves, and the juvenile form migrates through the peritoneal space to the liver, and then through the soft tissue of this organ into the bile ducts, where it achieves sexual maturity. The disease caused by the liver fluke, called fasciolosis, causes emaciation in animals, a drop in milk production, a drop in fertility, and may also cause the animal's death (Kadlubowski et al. 1999). It is thus a disease with great economic implications, causing great financial loss. Furthermore, an increasing number of human infections is noted, particularly in developing countries where it is supposed that every third citizen may be infected, and the more severe infections with the liver fluke may in fact be fatal

The liver fluke is perceived as one of the most significant veterinary parasitic problems in the world, and protective vaccination is becoming the most effective and economical medical intervention. The vaccine's efficacy is not only decided by its immunizing properties, but also its availability, generally a product of its price. Presently research also concentrates on lowering the cost of vaccine production.

Treatment is based on the application of anti-fluke drugs, and prevention on combating the snail, the intermediate host of the parasite, as well as on draining meadows and pastures. To date there is no effective vaccine, though many attempts have been made to find an appropriate fluke antigen, which would cause a strong immune response. To date, the antigens used in vaccination were antigens from the fluke itself (Wedrychowicz and Klockiewicz, Acta Parasitologica 1994, 39:173).

The fluke's migration through the tissues of the definitive host is made possible by secreted proteolytic enzymes, among these cysteine proteases. (Piacenza L. et al. Parasitol Int., 1998, 47S:266), which degrade the proteins of the extracellular matrix, first in the walls of the digestive tract, and later in the bile duct and liver. Thus, this is a key factor which facilitates the development of the parasite, but is also the first element of the parasite-host interaction. proteolytic enzymes play an important role in the life cycle of all parasites, since they allow them to invade the host's cells and tissues, they degrade elements of the immune system and hydrolyse the host's proteins as nutrients for the parasite. As a result of evolution, the enzymes became more specific, which influenced the adaptive success of parasites.

The cysteine protease belongs to a group of proteins called cathepsin L (*cathepsin like protein*)*.* Two heterogeneous forms of this protein are known, L1 and L2 (27 kDa and 29,5 kDa). cDNA of the cysteine protease has been used as an anti-fluke vaccine, with a successful immunization of rats (Kofta et al., Vaccine 2000, 18:2985).

Only a dozen years ago, it was thought that proteolytic enzymes secreted by the gastrodermal cells of the liver fluke were used solely to allow the penetration of host tissues and digestion of nutrients. It is presently know that cathepsin L can lyse antibodies in the hinge region, and protect the parasite against immunological attack. They also posses the ability to fragment fibrinogen, such that it forms a clot surrounding the fluke migrating through the host organism, and thus lessen the effectiveness of the immune response by inhibiting macrophage access. One of the cathepsins is also present in adult flukes, in the Melis' gland, where it participates in egg production. This means that the enzyme is secreted at every stage of the parasite's life.

Transgenesis has made it possible to use animal and plant organisms as bioreactors. Organisms obtained in this way produce antigen proteins, which induce a readiness state against invading pathogens in the immune system (Charon et al., "Animal Genetics", PWN, Warsaw, 2000). In vaccines of this type, the immunogen is a single antigen, such as a viral, bacterial or parasitic protein. Stimulation of the immune system is possible, when the protein retains its unchanged, native structure, which means that disease causing factors usually require and eucaryotic expression system. Thus their production in yeast or bacteria, even though cheaper and more efficient, is often unsuccessful due to the lack of appropriate protein-modifications.

Patent description number US6084156 (published 2000.07.04) describes a solution relating to plants producing lytic peptides. Except for description US6084156, patent descriptions US6448391 (published 2002.09.10) and US6018102 (published 2000.01.25) and US5955573 (published 1999.09.21) also describe solutions relating to the stabilizing combination of polypeptides with ubiquitin lytic peptide, as well as a method of manufacturing through the subcloning of a nucleic acid coding the sequence of a lytic peptide into a plasmid vector containing a promoter and a sequence coding the ubiquitin polypeptide, in which the ubiquitin polypeptide is bound with the 5' end of the sequence of the nucleic acid sequence of the lytic peptide undergoing translation as a fusion polypeptide. Descriptions US6018102 and US5955573 further describe solutions regarding gene constructs combining the ubiquitin lytic peptide, their protein products and their production and application.

Patent description US6306663 (published 2001.10.23) describes a solution relating to new compounds containing ubiquitin recognition elements and a protein binding factor. This invention also relates to the application of said compounds in order to modulate the level and/or level of activity of the target protein. These compounds are useful in application against infection, inflammation states, cancer and genetic diseases, as well as herbicides and insecticides.

Patent description US6068994 (published 2000.05.30) describes an invention relating to the expression of ubiquitin. The solution describes a method for conjugating ubiquitin to the expression of a gene, facilitating a controllable high level of production of heterologous proteins possessing destabilizing amino-acid residues in a terminal position. Ubiquitin fusion proteins undergoing expression in yeast are precisely cleaved *in vivo* through endogenous hydrolases specific for ubiquitin into heterologous yeast proteins such as human alfa-1-antitrypsin, human gamma-interferon and human HIV virus protein. which are initiated with destabilising residues. A synthetic vector containing a synthetic gene of monomeric yeast ubiquitin was constructed and underwent expression under the control of the yeast regulatory glucose promoter. This system may be used to increase the level of expression for proteins showing a low level of expression and for the production of proteins possessing destabilizing amino-acid residues in a terminal position.

In patent descriptions US 5847097 (published 1998.12.08) and US5646017 (published 1997.07.08) a method is presented for the production or modification of protein structure at the level of protein or gene in order to produce specific amino-termini *in vivo* or *in vitro.* These methods are based on the unnatural introduction of protein-ubiquitin conjugates and the determination that the half-life of the protein *in vivo* is a function of the N-terminal amino-acid of the protein.

Patent description US5620923 (published 1997.04.15) describes a method of manufacturing synthetic protein products containing approximately forty amino-acid residues lengthened by ubiquitin at the carboxyl end, undergoing expression in prokaryotic cells such as *E. coli.* This process may be applied in the production of peptides containing from 2 to around 40 amino-acid residues and is particularly applicable in the production of peptides containing from 5 to 40 amino-acid residues.

Patent description US 5494818 (published 1996.02.27) relates to a generic class of ubiquitin-specific proteases which specifically cleave groups near the C-end residue of ubiquitin in a ubiquitin fusion protein, regardless of size. More specifically, the invention relates to ubiquitin-specific proteases from a class isolated from a cell and isolated DNA sequences coding this class of protein.

Attempts to produce antigens for vaccines in mammal and insect cell cultures entailed excessive costs, a necessity to constantly control the synthesis and precise purification of the vaccines such that they did not constitute potential sources of allergens or factors causing auto-immune reactions.

Plant bioreactors are much cheaper and safer. Proteins produced in a plant cell from exogenous DNA possess an identical amino-acid sequence, undergo similar modifications, and acquire similar properties to those appearing during pathogenesis in the host organism. Transgenic plants possess the foreign DNA in all of their cells, which codes for the appropriate antigen, and produce that particular antigenic protein. The foreign gene inserted is passed on onto offspring.

Modified plants may be consumed in a raw state, which means no costs entailed by the purification of the antigen. Oral immunization seems the most acceptable due to the non-invasive and painless application, and efficacy in causing a systemic response (McGhee J.R., Lamm M.E., Strober W. 1999, w: Mucosal Immunology (Ogra P.L., Lamm M.E., Bienenstock J., Mestecky J., Strober W., McGhee J.R., red.), str. 485 - 505, Academic Press).

Patent descriptions US6281345 (published 2001.08.28), US6265198 (published 2001.07.24) describe pharmaceutical compositions containing nucleic acids, proteins, antibodies and inhibitors, as well as their application in protecting animals against diseases caused by gastrointestinal parasites.

Patent description US6066503 (published 2000.05.23) describes a solution relating to nucleotide sequences coding aminopeptidase enzymes of gastrointestinal parasites, their antigens and functional equivalents and their application as vaccinations against these parasites.

Patent description US5885814 (published 1999.03.23) presents a vaccine against gastrointestinal parasites, containing a serine protease isolated from the liver fluke.

Patent descriptions US6419923 (published 2002.07.22), US6365392 (published 2002.04.02), US5795768 (published 1998.08.18), US5792624 (published 1998.08.11), US5750391 (published 1998.05.12) and US5691186 (published 1997.11.25) describe methods of obtaining the cysteine protease protein, antibodies, nematode cysteine protease inhibitors and their application in pharmaceutical compositions for the protection of animals against diseases caused by gastrointestinal parasites, and isolated, recombined cells expressing them. In description US6365392 the composition containing particular, isolated sections of nucleic acid is a recombined viral vaccine.

Patent description US5863775 (published 1999.01.26) describes a method for combating gastrointestinal parasites in animals which a definitive hosts, which is based on the oral administration of anti-parasite protein, in the form of a drug or foodstuff, such that the protein reaches the parasite. The anti-parasite protein may be an inhibitor of the parasite's enzyme, such as a digestive enzyme inhibitor like a cysteine protease inhibitor. According to this solution, the anti-parasite protein is a protein which undergoes expression in transgenic maize and rice, which constitute animal fodder.

Despite the above described preliminary research devoted to the delivery of a liver fluke vaccine, a need still exists for effective tools for the facile production of efficacious vaccines immunizing against these parasites.

The goal of the present invention is the delivery of means which could be used to obtain a vaccine against the liver fluke. A particular goal of the invention is to produce chimaeric proteins, which would contain selected antigens from the cysteine protease isolated from *Fasciola hepatitca* and which could be used for vaccination against the liver fluke, as well as tools facilitating their expression, particularly in plant systems. A particular goal of the invention is to produce the means to obtain a transgenic plant, which could be applied to the production of edible vaccines against the liver fluke.

Unexpectedly, the embodiment of the described goal, and the solution of the described technological problems arising with proteins produced in cells on the basis of exogenous DNA have been achieved in the present invention.

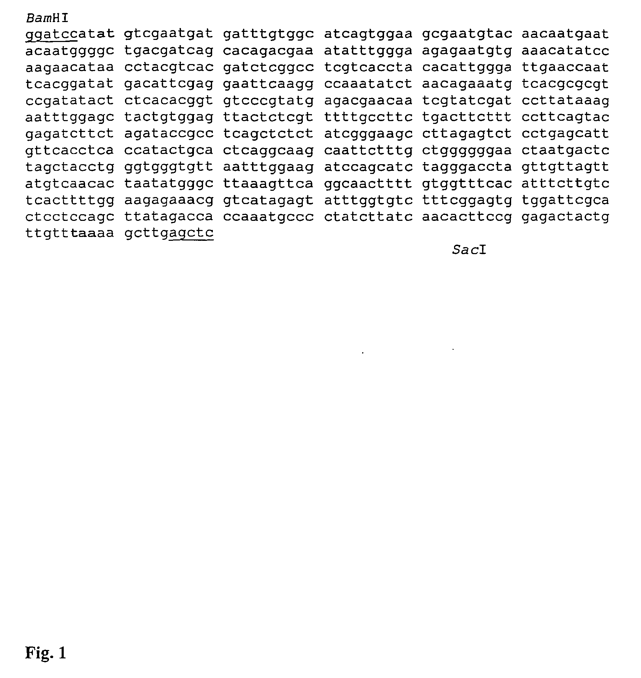

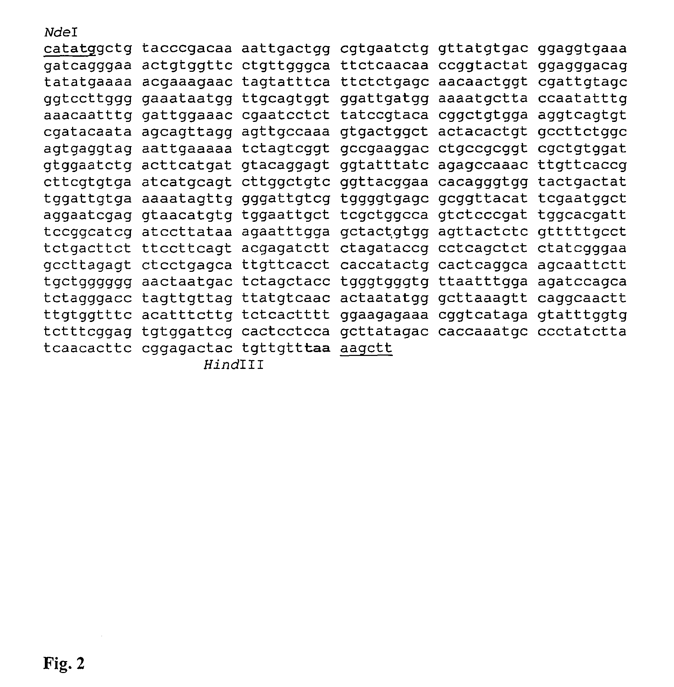

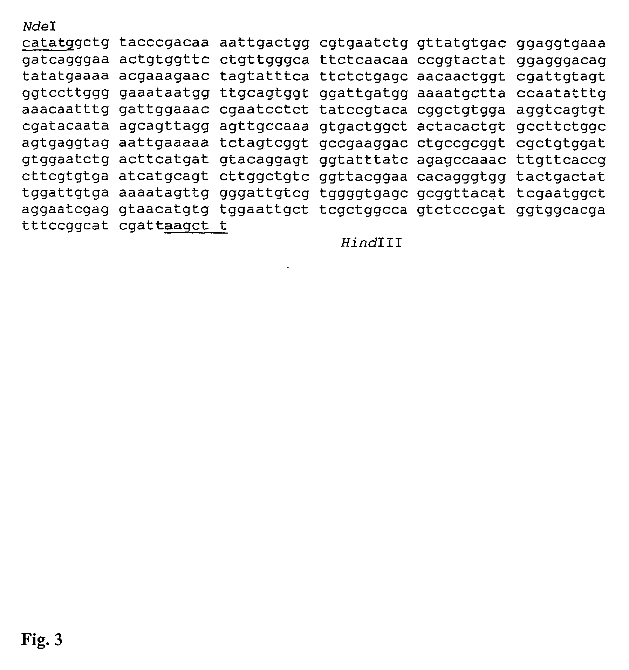

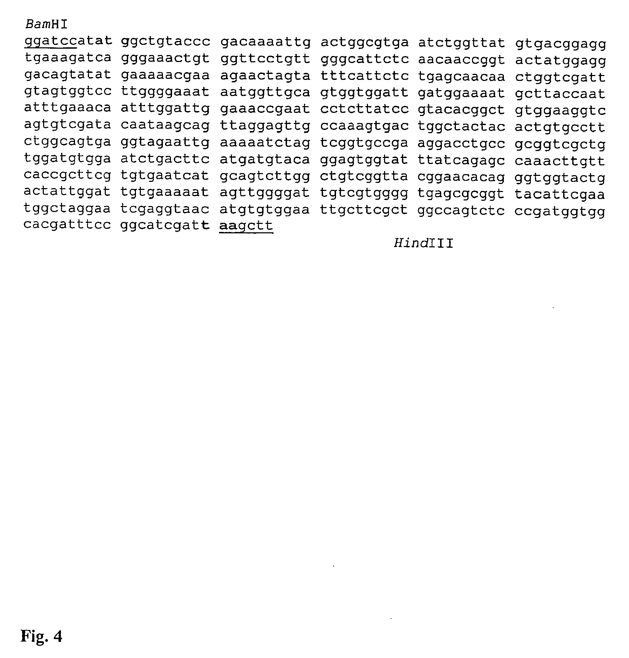

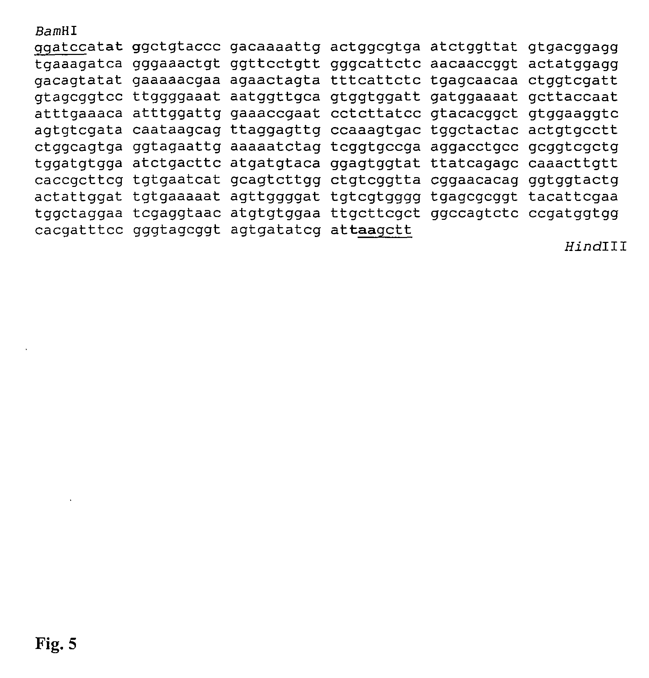

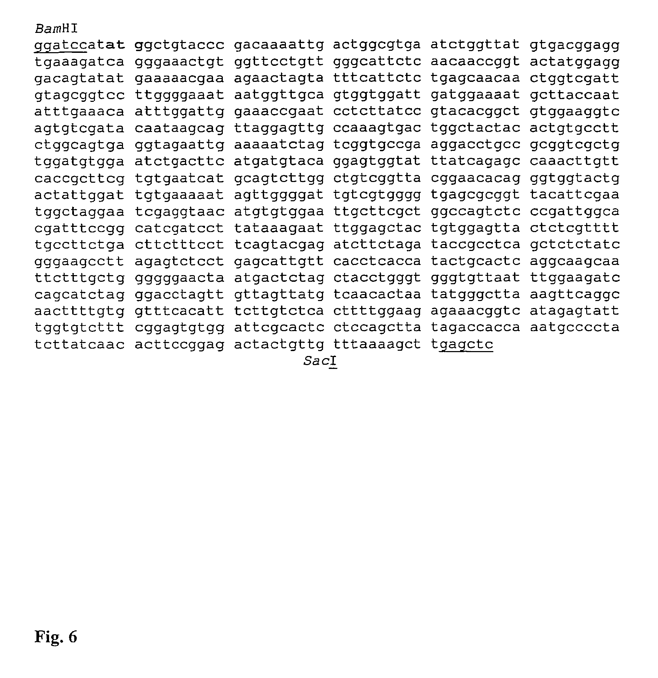

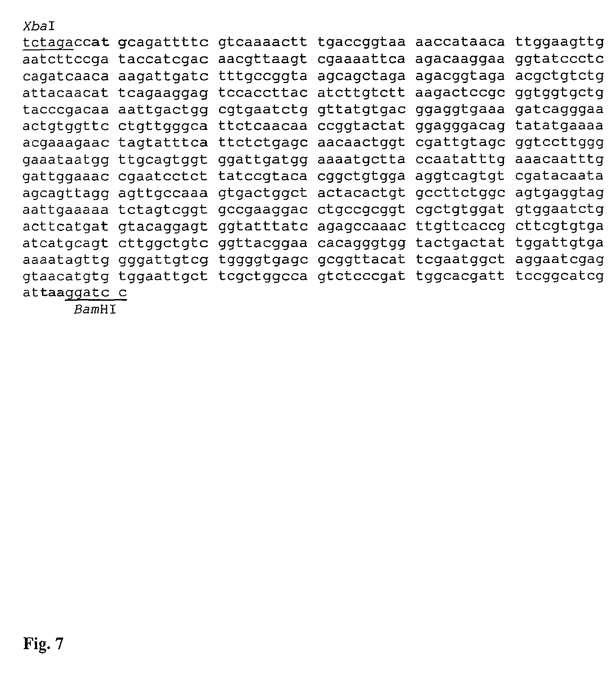

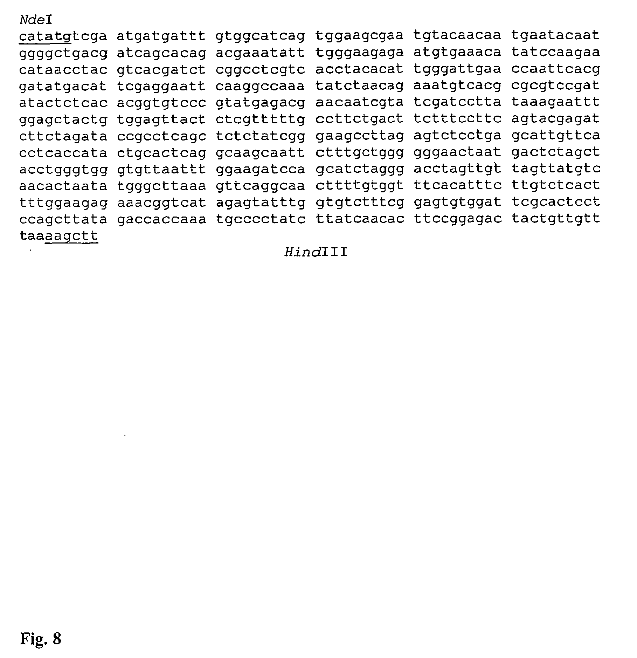

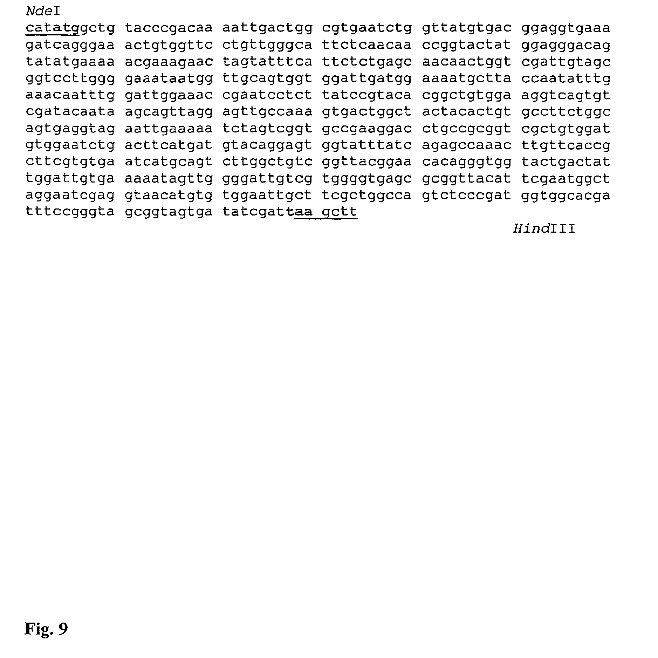

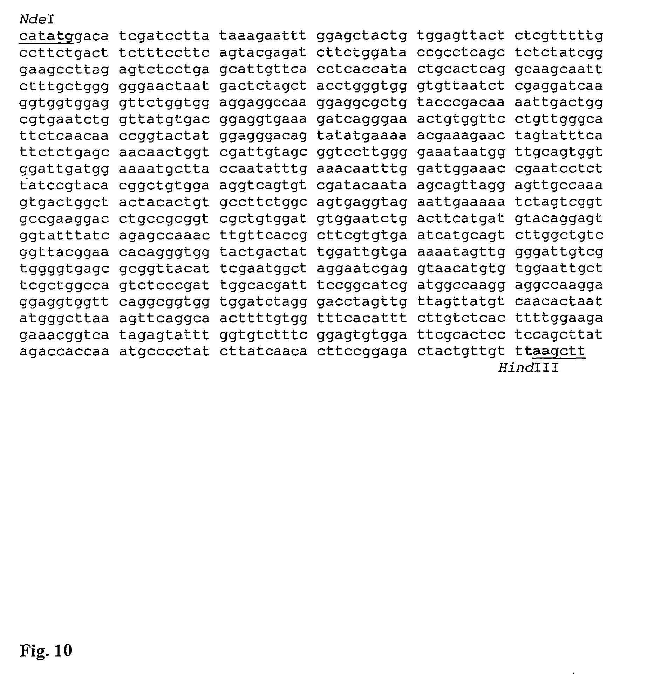

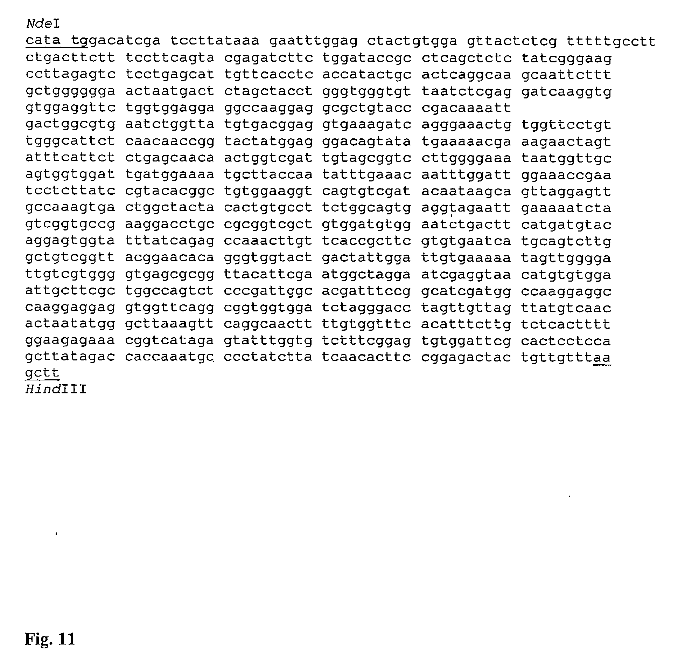

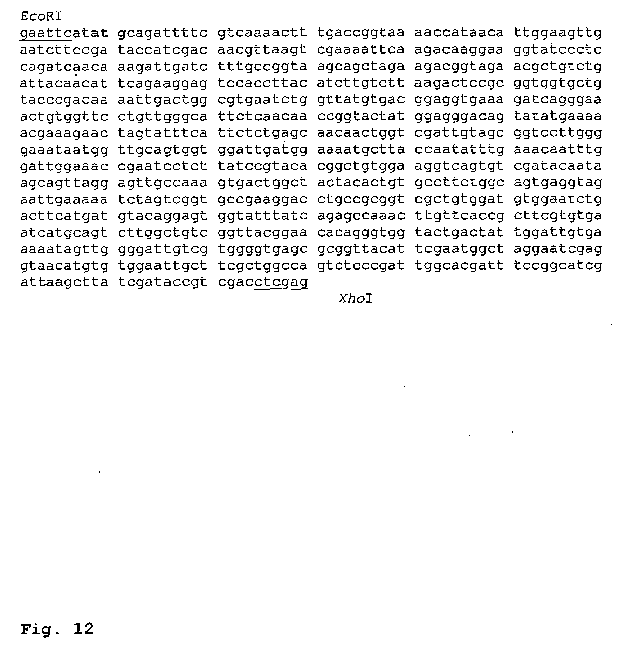

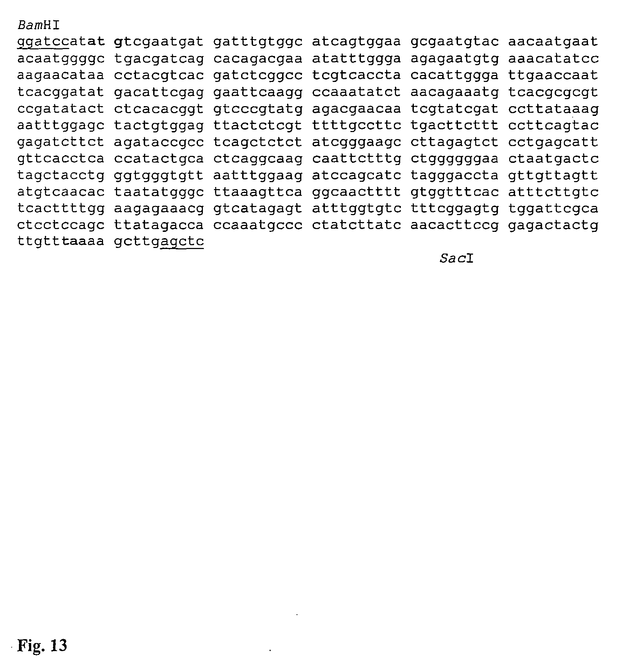

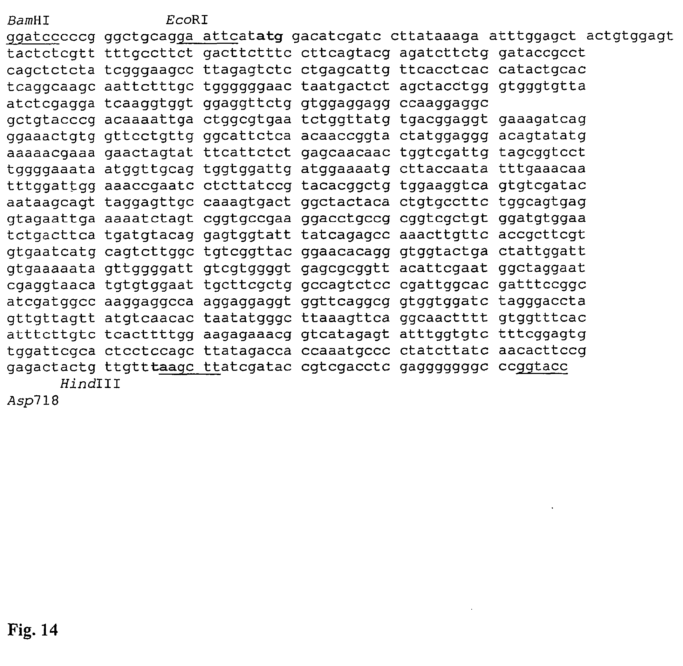

The present invention relates to a nucleic acid molecule coding a chimaeric protein under the control of the constitutive promotor 35S CaMV characterized in that, the sequence coding for the chimeric protein consists of one of the following sequences:
- a nucleotide sequence coding the amino acid sequence of the catalytic domain of the liver fluke cysteine protease fused to the sequence coding the core protein *HBc,*
- a nucleotide sequence coding the amino acid sequence of the leading domain of the liver fluke cysteine protease fused to the sequence coding the core protein *HBc,*
- a nucleotide sequence coding the amino acid sequence of the catalytic domain of the liver fluke cysteine protease fused to the sequence coding the core protein *HBc,* and glycine residues, or
- a nucleotide sequence coding the amino acid sequence of the catalytic domain of the liver fluke cysteine protease fused to the nucleotide sequence coding ubiquitin.
Preferentially, the sequence for the chimeric protein has been selected from among the sequences presented in Figs. 1-14.

The next topic of the present invention is a plant cell characterized in that it comprises a nucleic acid sequence as defined above.

The next topic of the present invention is a method for the production of a chimaeric protein which encompasses the introduction of a vector into cells of *Agrobacterium tumefaciens* using electroporation, transformation of plants with a strain of bacteria comprising this vector, and the expression of the chimaeric protein, wherein the vector consists of a construct comprising one of the sequences as defined above.

The next topic of the present invention is a method for the production of a transgenic plant, which encompasses the introduction of a vector into cells of *Agrobacterium tumefaciens* using electroporation, transformation of plants with a strain of bacteria consisting of this vector, and regeneration of the plants, wherein the vector consists of a construct comprising one of the sequences one of the sequences as defined above.

The next topic of the present invention is a transgenic plant which possesses cells transformed with a construct comprising a nucleotide sequence, characterized in that it possesses cells transformed with a construct consisting of one of the sequences as defined above.

The next topic of the present invention is a Use of a transgenic plant transformed with a construct comprising a nucleotide sequence coding the amino acid sequence, characterized in that the chimaeric protein consists of one of the sequences as defined above.

The next topic of the present invention is a pharmaceutical composition containing a plant cell comprising a nucleotide sequence coding the amino acid sequence of a chimeric protein or a transgenic plant possessing cells transformed with a nucleotide sequence coding the amino acid sequence of a chimaeric protein or its tissues or their derivative products and possibly a pharmaceutically allowable carrier, characterized in that the amino acid sequence of said chimaeric protein consists of one of the sequences as defined above. Preferentially, a pharmaceutical composition is a liophilisate.

The next topic of the present invention is a vaccine against liver fluke containing a plant cell comprising a nucleotide sequence coding the amino acid sequence of a chimeric protein or a transgenic plant possessing cells transformed with a nucleotide sequence coding the amino acid sequence of a chimaeric protein or its tissues or their derivative products and possibly a pharmaceutically allowable carrier, characterized in that the amino acid sequence of said chimaeric protein consists of one of the sequences as defined above.
Preferentially, the vaccine is a liophilisate.
Preferentially, the vaccine is an oral vaccine.

The next topic of the present invention is a plant cell as defined above or a transgenic plant as defined above for use in the induction of immunity against liver fluke infections in mammals.
Preferentially, the said treatment is given orally.

According to the present invention, the sequence coding the proteases have been divided into regions coding the catalytic domain and the leading domain of the enzyme, since the usage of the entire sequence coding the cysteine protease could cause the formation of the fully active enzyme with its hydrolytic properties, which could cause developmental problems in plants. In a particular embodiment of the present invention, these fragments have been fused to the sequence coding the HBc core protein (*hepatitis B core*) of the *HBV virus.* Such chimaeric genes have been placed under the control of the constitutive promoter 35S CaMV in the binary vector ROK2 resulting in pMKCROK2 and pMLCROK2. Additionally, vectors were produced using the catalytic domain, fused to the *HBc* core protein along with several glycine residues. The catalytic sequence has also been fused with ubiquitin. These plasmids were introduced into plants using procedures facilitating their integration with the plant genome.

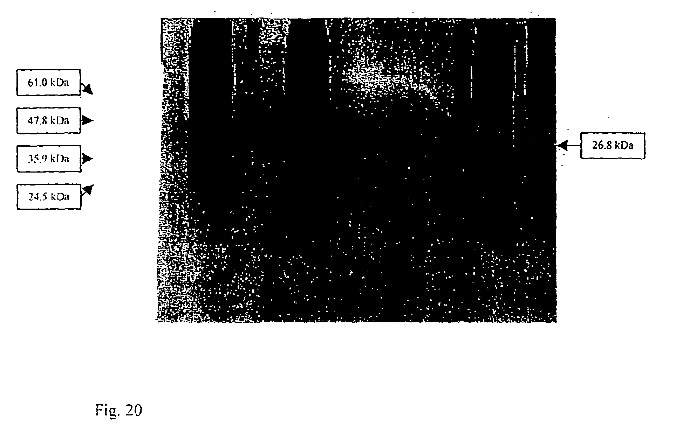

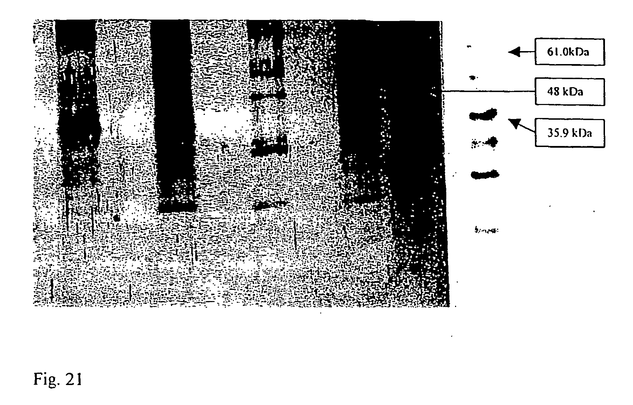

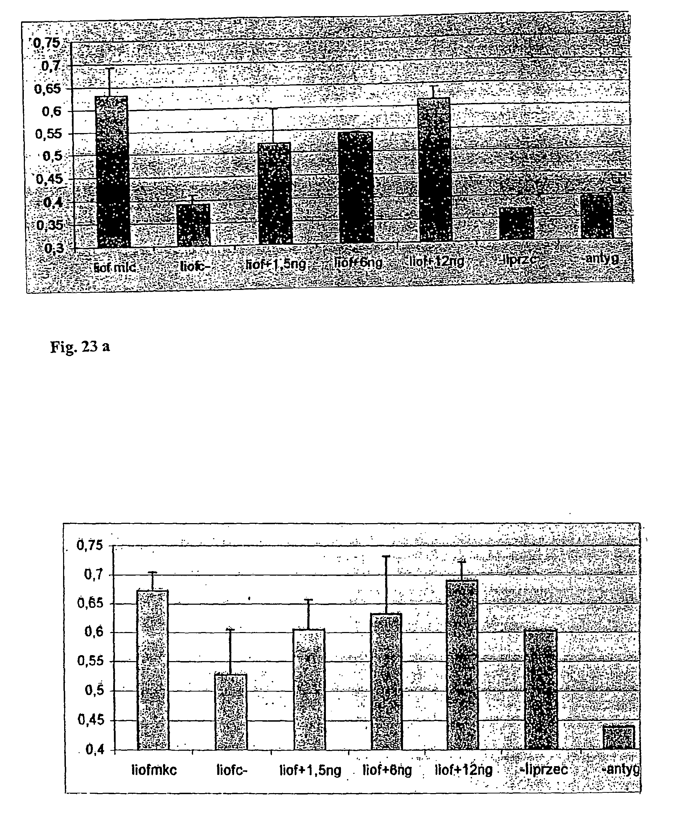

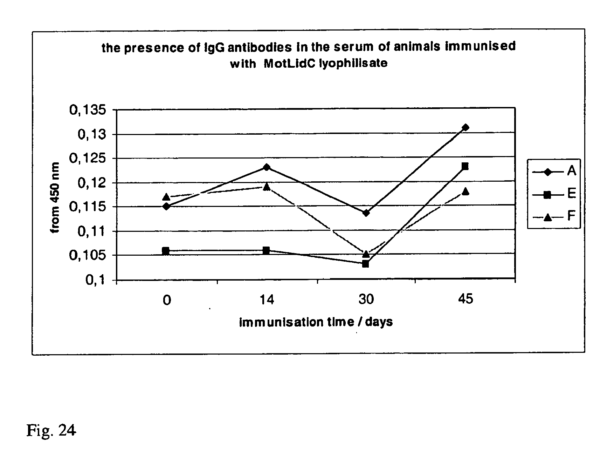

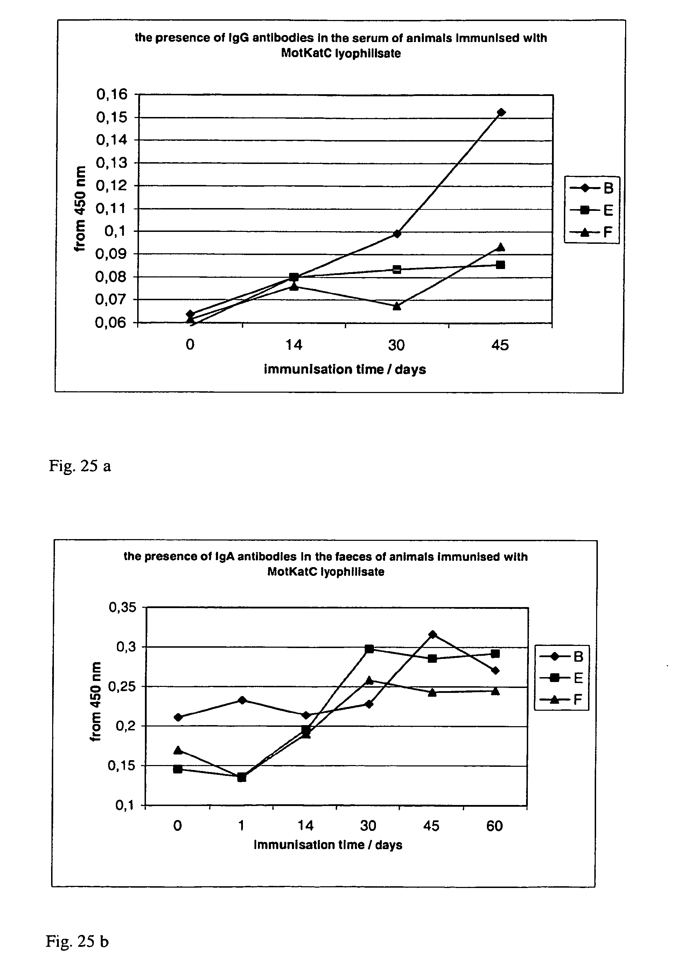

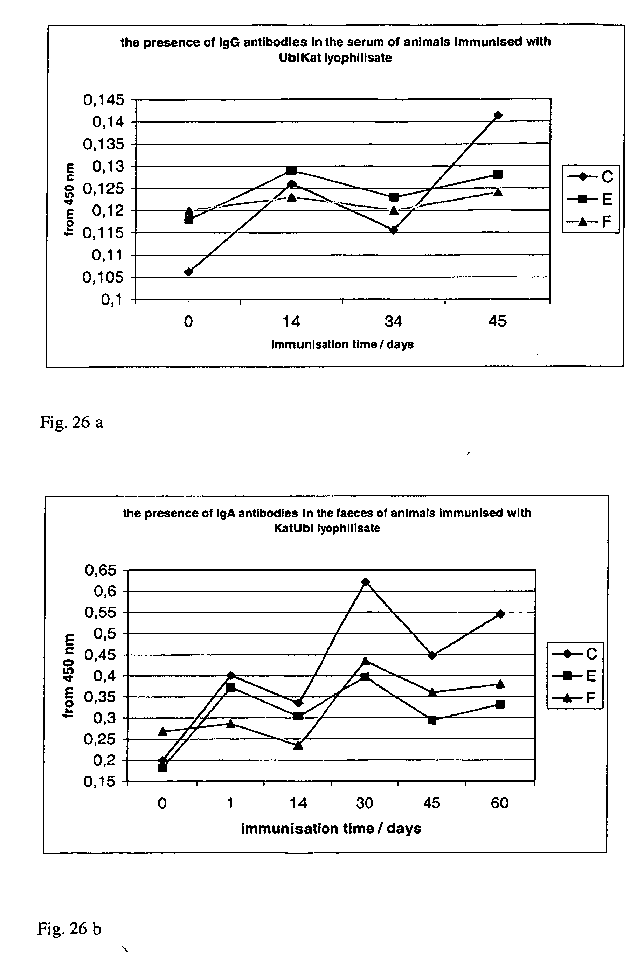

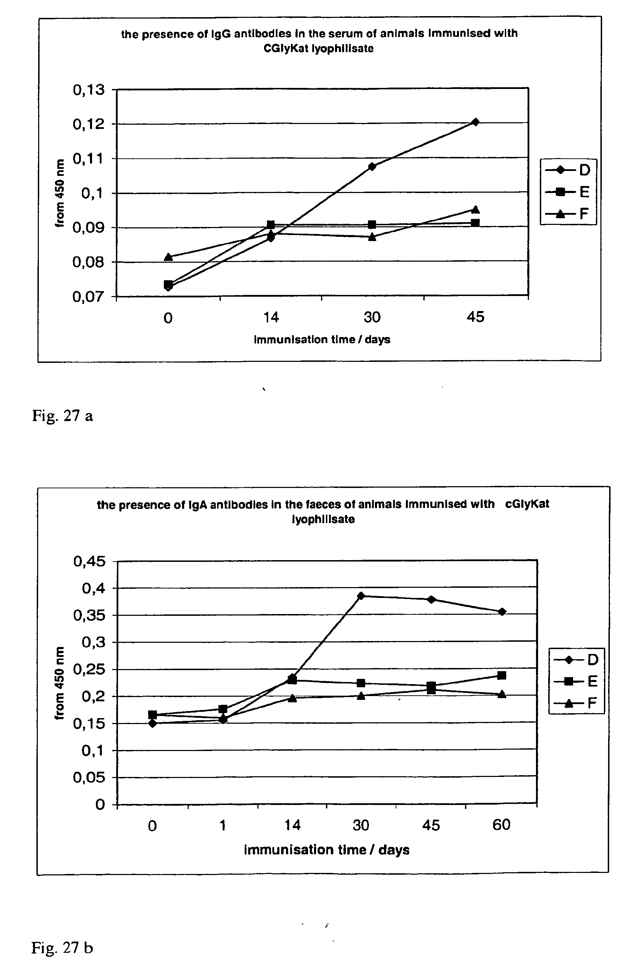

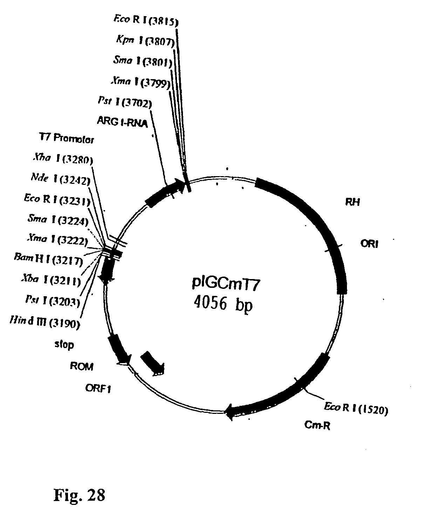

The attached figures facilitate a better understanding of the nature of the present invention.
Figure 1 presents the sequence coding the hybrid protein 'core HBV fragment::liver fluke cysteine protease leading domain'. This fragment was introduced into the ROK2 vector into places arising from digestion with BamHI and SacI restriction nucleases.
Figure 2 presents the sequence coding the hybrid protein 'core HBV fragment ::liver fluke cysteine protease catalytic domain'. The fragment was introduced into the pIGCmT7 vector into places arising from digestion with NdeI and HindIII restriction nucleases.
Figure 3 presents the sequence coding the liver fluke cysteine protease catalytic domain. The fragment was introduced into the pIGCmT7 vector into places arising from digestion with NdeI and HindIII restriction nucleases.
Figure 4 presents the sequence coding the liver fluke cysteine protease catalytic domain. The fragment was introduced into the Bluescript SK(-) vector into places arising from digestion with BamHI and HindIII restriction nucleases.
Figure 5 presents the sequence coding the liver fluke cysteine protease catalytic domain. The fragment was introduced into the Bluescript SK(-) vector into places arising from digestion with BamHI and HindIII restriction nucleases.
Figure 6 presents the sequence coding the hybrid protein 'core HBV fragment ::liver fluke cysteine protease catalytic domain'. The fragment was introduced into the ROK2 vector into places arising from digestion with BamHI and SacI restriction nucleases.
Figure 7 presents the sequence coding the hybrid protein 'ubiquitin::liver fluke cysteine protease catalytic domain'. The fragment was introduced into the ROK2 vector into places arising from digestion with XbaI and BamHI restriction nucleases.
Figure 8 presents the sequence coding the hybrid protein 'core HBV fragment ::liver fluke cysteine protease leading domain' The fragment was introduced into the pIGCmT7 vector into places arising from digestion with NdeI and HindIII restriction nucleases.
Figure 9 presents the sequence coding the liver fluke cysteine protease catalytic domain. The fragment was introduced into the pIGCmT7 vector into places arising from digestion with NdeI and HindIII restriction nucleases.
Figure 10 presents the sequence coding the hybrid protein 'core HBV fragment ::liver fluke cysteine protease catalytic domain'. The fragment was introduced into the pIGCmT7 vector into places arising from digestion with NdeI and HindIII restriction nucleases.
Figure 11 presents the sequence coding the hybrid protein 'core HBV fragment ::liver fluke cysteine protease catalytic domain'. The fragment was introduced into the Bluescript SK (-) vector into places arising from digestion with EcoRI and HindIII restriction nucleases.
Figure 12 presents the sequence coding the hybrid protein 'ubiquitin ::liver fluke cysteine protease catalytic domain' The fragment was introduced into the Bluescript SK (-) vector into places arising from digestion with EcoRI and XhoI restriction nucleases..
Figure 13 presents the sequence coding the hybrid protein 'core HBV fragment ::liver fluke cysteine protease leading domain'. The fragment was introduced into the ROK2 vector into places arising from digestion with BamHI and SacI restriction nucleases.
Figure 14 presents the sequence coding the hybrid protein 'core HBV fragment ::liver fluke cysteine protease catalytic domain' The fragment was introduced into the ROK2 vector into places arising from digestion with BamHI and Asp718. The cloned fragment contains a sequence from the Bluescript SK (-) vector at the beginning (to the EcoRI restriction site) and at the end (from the HindIII restriction site).
Figure 15 presents an analysis of transformants by PCR using specific oligonucleotide primers. Confirmation of transgenes in the vectors: a - pMKCROK2, *A*. *tumefaciens* LBA4404pMKCROK2, b - pMLCROK2, *A*. *tumefaciens* LBA4404pMLCROK2, c - *A. tumefaciens* EHA105MOTUBIKatROK, d - *A*. *tumefaciens* EHA105MOTGlyKatROK;
Figure 15a: 1-8. A.t LBA4404 pMKCROK2, 9. negative control, 10. DNA standard 1 kbp (Sigma), *E. coli* pMKCROK2, 18. positive control, pMKCROK2;
Figure 15b: 1-5. A.tLBA4404 pMLCROK2, 6-8. *E. coli* pMLCROK2, 9. DNA standard 1kbp (Sigma), 10 positive control pMLCROK2;

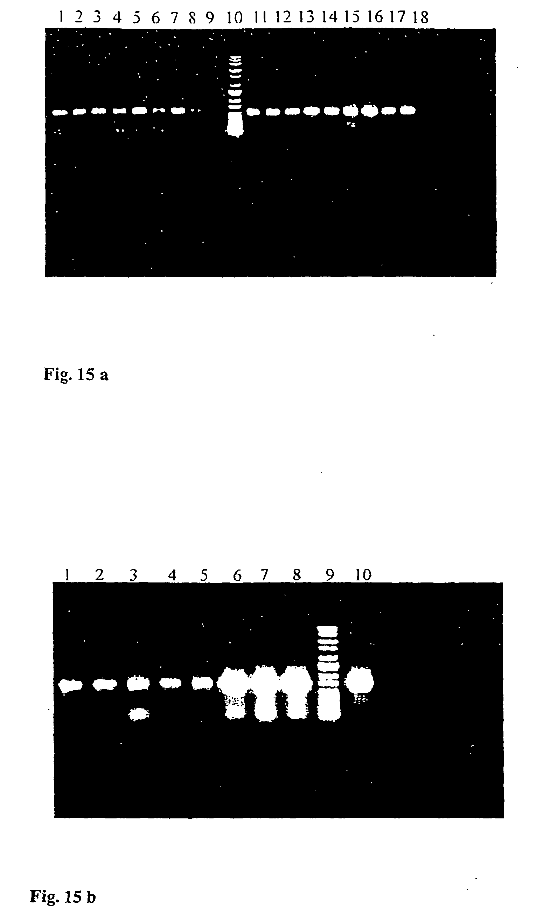

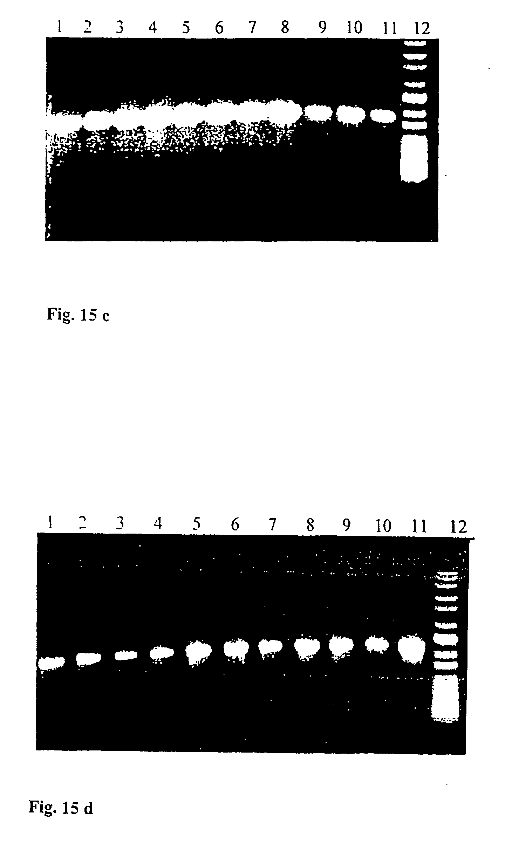

Figure 15 c: 1-10. *A*. *tumefaciens,* EHA105MOTUBIKatROK, 11. positive control pMOTUBIKatROK, 12. DNA standard 1 kbp (Sigma);
Figure 15 d: 1-10 *A*. *tumefaciens* EHA105MOTGlyKatROK, 11 positive control MOTGlyKatROK, 12. DNA standard 1 kbp (Sigma)

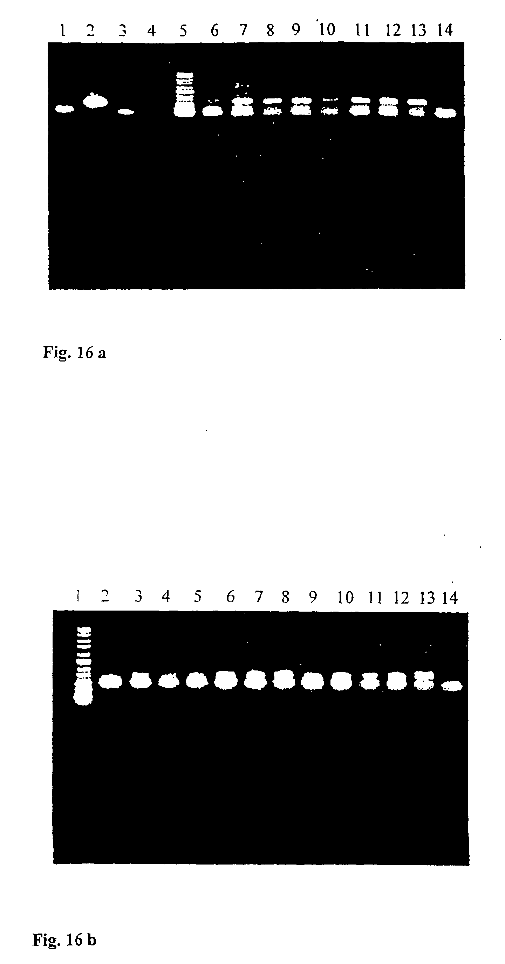

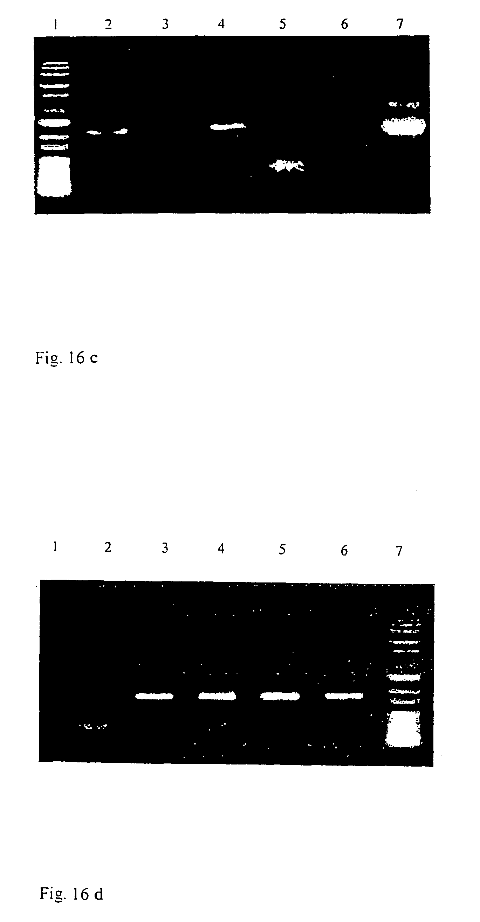

Figure 16 presents a PCR analysis with regards to the MotKat hybrid sequence - C, MotLid - C, MotKatGly - C, MotKatUbi. The primers MotL5 and MotL3 specific for the leading domain were used; Mot5 and MotK3 for the sequence of the catalytic domain; MOTGly5 and MOTGly3 for the domain containing the glycine bridge, as well as UBIROCK and MOTKATRO for the construct containing the ubiquitin sequence.
Figure 16a: untransformed lettuce 1 - negative control, 2 positive control pMKCROK, 3. water - negative control, 5. DNA standard 1kbp (Sigma), 6-14. DNA samples from transgenic plants;
Figure 16 b: 1- DNA standard 1kbp (Sigma), 2-12 DNA samples from transgenic plants; positive control pMLCROK; woda - negative control
Figure 16 c: 1- DNA standard 1kbp (Sigma), 2-6 DNA samples from transgenic plants; positive control p35SMOTGlyKatRok;
Figure 16 d: 1-4 DNA samples from transgenic plants, 5 positive control p35SMOTUBIROK, 6 DNA standard 1kbp (Sigma);

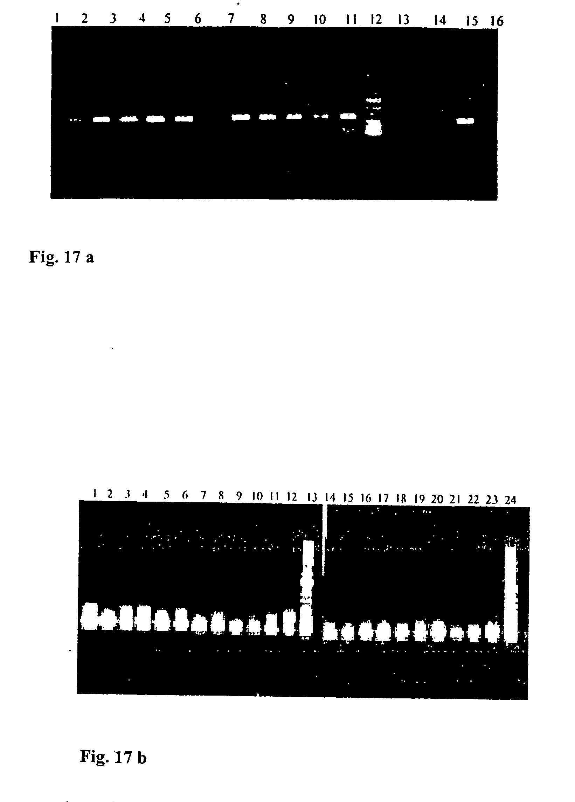

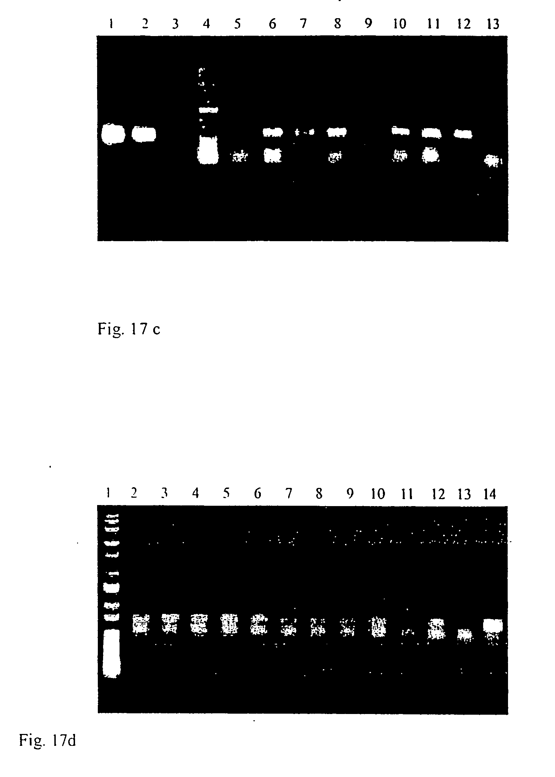

Figure 17 presents PCR analysis of the T1 generation of transformed lettuce plants for the presence of transgenes: a- MotKat -C, b - MotLid - C, c - MotKatGly - C, d - MotKatUbi;
Figure 17 a: 1-14. genotype A1, 15. positive control, 16. negative control, 12 . DNA standard 1 kbp;
Figure 17b 1-12. genotype A1, 14-20. genotype A2, 22. positive control, 23. negative control, 13,24. DNA standard 1 kbp.;
Figure 17c 1-2. positive control, 3. negative control, 4. DNA standard 1 kbp., 5-13. genotype A1;
Figure 17d 1. DNA standard 1 kbp., 2-12. genotype A1, , 13. negative control, 14. positive control;

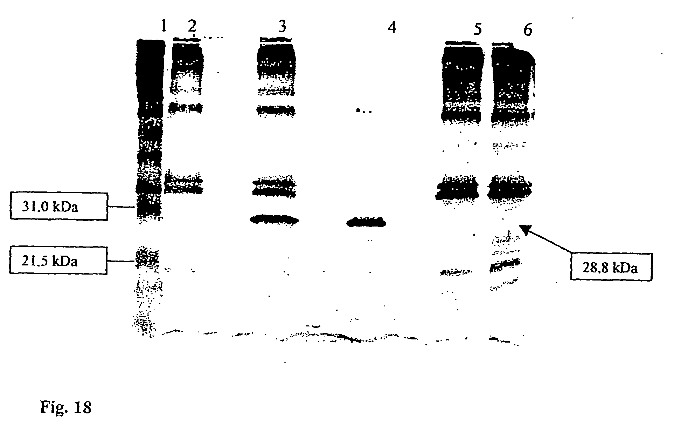

Figure 18 presents a Western blot of protein extract from lyophilised lettuce, transformed with the pMLCROK2 vector, where the paths represent, in turn:
   1. protein standard (Promega)
   2. protein extract of the control lyophilysate, untransformed
   3. protein extract of the control lyophilysate, untransformed with an addition of antigen (100 ng)
   4. positive control, purified hybrid protein containing the cysteine protease leading domain fused to protein c of *HBV* (100 ng)
   5,6. protein extract of the transgenic lyophilysate, the product marked is the antigen protein, 28,8 kDa in size.

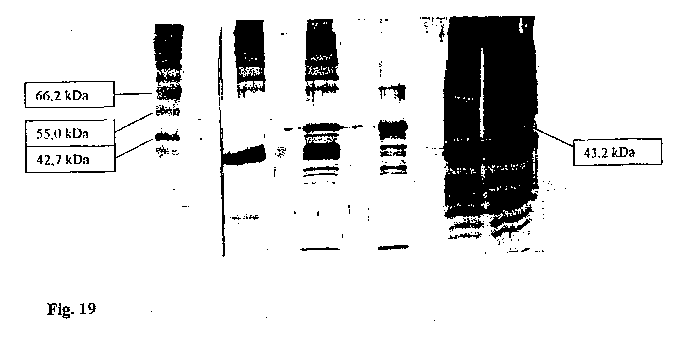

Figure 19 presents a Western blot of protein extract from lyophilised lettuce, transformed with the pMLCROK2 vector, where the paths represent, in turn:
   1. protein standard (Promega)
   2. protein extract of the control lyophilysate, untransformed
   3. protein extract from a control plant, untransformed with an addition of antigen (100 ng)
   4. positive control, purified hybrid protein containing the cysteine protease catalytic domain fused to protein c of *HBV* (100 ng)
   5,6. protein extract of the transgenic lyophilysate, the product marked is the antigen protein, 43,2 kDa in size
Figure 20 presents a Western blot of protein extract from lyophilysed lettuce, transformed with the p35SMOTUBIROK vector, where the paths represent, in turn:
   5. protein standard (Invitrogen)
   6. protein extract of the control lyophilysate, untransformed
   7. protein extract of the control lyophilysate, untransformed with an addition of antigen (100 ng)
   8. positive control, purified hybrid protein containing the cysteine protease catalytic domain (100 ng)
   5,6. protein extract of the transgenic lyophilysate, transgenic lyophilysate, the product marked is the antigen protein, 26,8 kDa in size
Figure 21 a Western blot of protein extract from lyophilised lettuce, transformed with the p35SMOTGlyKatRok vector, where the paths represent, in turn:
   9. protein standard (Invitrogen)
   10. protein extract of the control lyophilysate, untransformed
   11. protein extract of the control lyophilysate, untransformed with an addition of antigen (100 ng)
   12. positive control, purified hybrid protein containing the cysteine protease catalytic domain (100 ng)
   5,6. protein extract of the transgenic lyophilysate, he product marked is the antigen protein, 48 kDa in size.

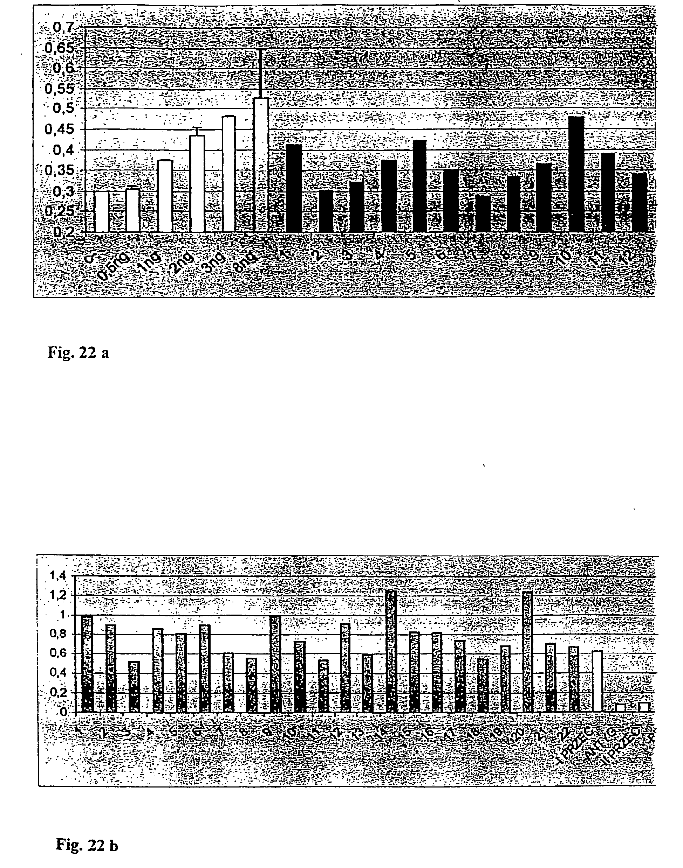

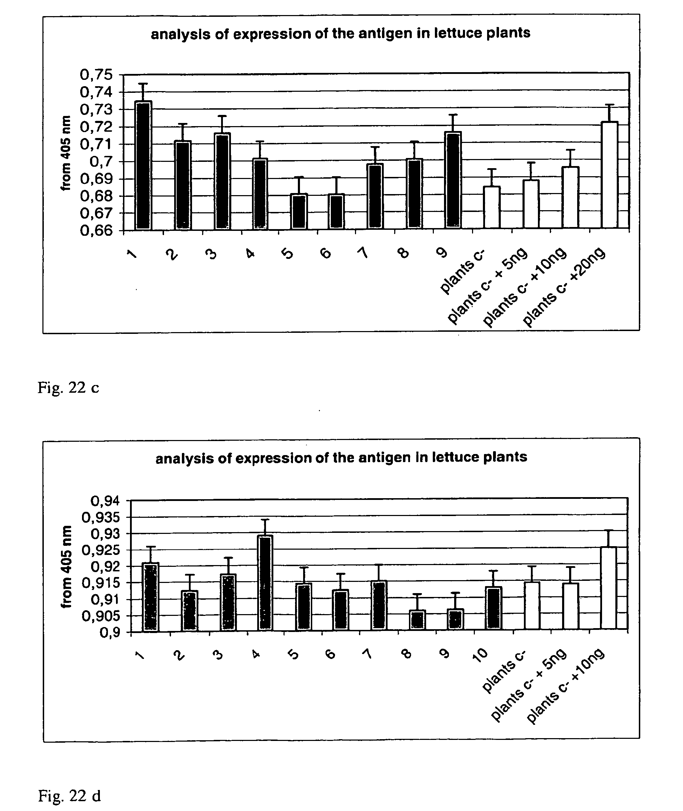

Figure 22 presents an ELISA immunoenzymatic assay to confirm expression of the antigen in lettuce plants:
   a) control plants, into which the cysteine protease leading sequence fused with protein c of HBV was introduced, where:
      c- - protein extract from non-transgenic lettuce
      - protein extract from non-transgenic lettuce with an addition of antigen 0,5 ng, 1 ng, 2 ng, 3 ng, 8ng
      1-12 - protein extract from transgenic lettuce containing the cysteine protease leading fragment fused to protein c of HBV
   b) control plants, into which the cysteine protease catalytic sequence fused with protein c of HBV was introduced, where:
      1-22 - protein extract from transgenic lettuce containing the cysteine protease catalytic sequence fused with protein c of HBV, protein extract from non-transgenic lettuce
   c) control plants, into which the cysteine protease catalytic sequence fused with protein c of HBV with the glycine bridge, was introduced where:
      1-9- protein extract from transgenic lettuce containing the cysteine protease catalytic sequence fused with protein c of HBV with the glycine bridge
      c- - protein extract from non-transgenic lettuce
         - protein extract from non-transgenic lettuce with an addition of antigen 5 ng, 10 ng, 20 ng
   d) control plants, into which the cysteine protease catalytic sequence fused with ubiquitin, where:
      1-10 - protein extract from transgenic lettuce containing fragment the cysteine protease catalytic sequence fused with ubiquitin
      c- - protein extract from non-transgenic lettuce
      - protein extract from non-transgenic lettuce with an addition of antigen 5 ng, 10 ng
Figure 23 presents an ELISA immunoenzymatic assay to confirm expression of the antigen in lettuce lyophilisate:
   a) analysis of lyophilisate containing the sequence of the cysteine protease leading sequence fused with protein c of HBV was introduced, where:
      - lyophilisate of transgenic lettuce
      - lyophilisate of non-transgenic lettuce
      - lyophilisate of non-transgenic lettuce with an addition of antigen 1,5ng, 6 ng, 12 ng
      - II antibody - negative control, system lacking the II polyclonal antibody anti-L5
   b) analysis of lyophilisate containing the sequence of the cysteine protease catalytic sequence fused with protein c of HBV was introduced, where:
      - lyophilisate of transgenic lettuce
      - lyophylisate of non-transgenic lettuce
      - lyophylisate of non-transgenic lettuce with an addition of antigen 1,5ng, 6ng, 12 ng
      - II antibody - negative control, system lacking the II polyclonal antibody anti-K4
Figure 24 presents an ELISA immunoenzymatic assay of: a - laboratory animal serum (mice) for the presence of IgG antibodies specific for the administered liver fluke antigen, the hybrid protein composed of the cysteine protease leading sequence fused with protein c of HBV, where:
   - m A mice fed transgenic lettuce
   - m E mice fed non-transgenic lettuce
   - m F mice given phosphate buffer
Figure 25 presents an ELISA immunoenzymatic assay of: a - serum and b - faeces of laboratory animals (mice) for the presence of antibodies, IgG (serum) and IgA (faeces) specific for the administered liver fluke antigen, the hybrid protein composed of the cysteine protease catalytic domain fused with the capsid protein of HBV, where:
   - m B mice fed transgenic lettuce
   - m E mice fed non-transgenic lettuce
   - m F mice given phosphate buffer
Figure 26 presents an ELISA immunoenzymatic assay of: a - serum and b - faeces of laboratory animals (mice) for the presence of antibodies, IgG (serum) and IgA (faeces) specific for the administered liver fluke antigen, the hybrid protein composed of the cysteine protease catalytic domain fused with the ubiquitin protein, where:
   - m C mice fed transgenic lettuce
   - m E mice fed non-transgenic lettuce
   - m F mice given phosphate buffer
Figure 27 presents an ELISA immunoenzymatic assay of: a - serum and b - faeces of laboratory animals (mice) for the presence of antibodies, IgG (serum) and IgA (faeces) specific for the administered liver fluke antigen, the hybrid protein composed of the cysteine protease catalytic domain fused with the capsid protein of HBV and glycine residues, where:
   - m D mice fed transgenic lettuce
   - m E mice fed non-transgenic lettuce
   - m F mice given phosphate buffer
Figure 28 presents the bacterial expression plasmid pIGCmT7 (4056 bp), which was designed based on the pIGDM1*ⁱ* plasmid, which is a plasmid belonging to the ColE1 group of *Enterobacter agglomerans.* The pIGCmT7 plasmid was produced through the introduction of a resistance gene to chloramphenicol, a polylinker along with a T7 RNA polymerase promoter and a sequence coding a transcription stop codon from phage T7, where:
   ARG t-RNA - arginine-tRNA gene for the AGA and AGG codons
   ORI - origin of replication for the pPIGDM1 plasmid
   Cm-R - chloramphenicol resistance gene
   T7 - promoter
   stop - transcription stop.

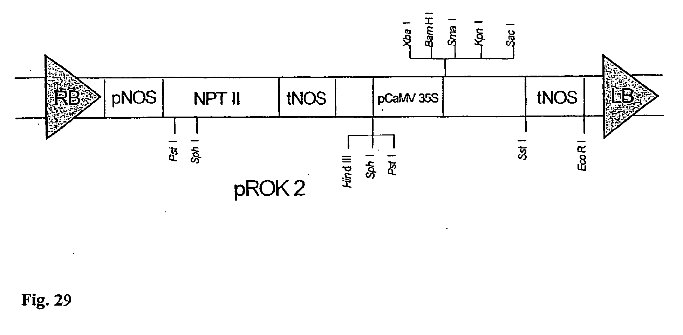

Figure 29 presents the plant expression plasmid, ROK2, where:
   RB - right flanking sequence
   LB - left flanking sequence
   pNOS - Nos promoter
   NPT II (Canamycin R) - canamycin resistance gene
   tNOS - Nos terminator
   pCaMV 35S - cauliflower mosaic virus promoter

Below are example embodiments of the above defined invention.

### Embodiment 1. Construction of vectors for plant transformation

The first variant related to the construction of the pMKC35SROK vector containing the sequence of the cysteine protease catalytic domain fused with the sequence coding the *HBc* protein of *HBV.*
The second variant related to the construction of the pMLC35SROK vector containing the sequence of the cysteine protease leading domain fused with the sequence coding the *HBc* protein of *HBV.*
The third variant related to the construction of the p35SMOTKatUBIROK vector containing the sequence of the cysteine protease catalytic domain fused with the sequence coding ubiquitin.
The fourth variant related to the construction of the p35SMOTGlyKatROK containing the sequence of the cysteine protease catalytic domain fused with the sequence coding ubiquitin. and glycine residues

Bacteria suspended in glicerol and stored in an ultrafreezer (temperature of - 70° C) were defrosted in an icebath. To bacteria prepared in such fashion 1 to 20 ng of plasmid DNA was added, gently stirred and incubated on ice for 2 - 6 min. Next, the bacteria were transferred to a chilled cuvette for electroporation. The cuvette was placed in the electroporator and current was applied. The electroporation was performed at 25 µF and 2500 V. Following electroporation, the bacteria were suspended in 1ml of SOC medium and transferred into flasks. The bacteria were shaken for 1 - 2 hrs. at 28°C, 50 - 60 rpm. Following incubation, the suspension was inoculated into YEB media with the antibiotics: rifampicine 100 mg/l and canamycin 50 mg/l and cultured for 2 days at 28°C.
A preliminary selection of transformed cells was made in a medium containing the selection factors rifampicine 100 mg/l and canamycin 50 mg/l. The second was a visualisation of the region of the introduced DNA through amplifying it using PCR (on boiled *A*. *tumefaciens* cells for 10 min. in 150 µl of water) and specific oligonucleotide primers.

The grown agrobacteria were inoculated into 10ml of liquid YEB medium with the selection factors: canamycin 50mg/l, rifampicine 100mg/l ⁱ in Erlenmayer flasks and cultured at 28°C. Following 16 - 20 hours of culturing the suspension of agrobacteria was inoculated onto fresh YEB ^{K50,R100} medium 1:100 and further cultured at 28°C until the bacteria attained the logarithmic stage of growth. The bacterial growth phase was estimated based on OD of the suspension at a 600 nm wavelength.

### Embodiment 2. Transformation and production of transgenic plants

**Table 1. Experimental variants performed**

| **Experimental variant** | Strain of *Agrobacterium* | *Lettuce variety* |
|---|---|---|
| LT-12 | *A. tumefaciens* LBA4404 pMKC35SROK | Syrena |
| LT-13 | *A. tumefaciens* LBA4404 pMLC35SROK | Bipp Burple |
| LT-14 | *A. tumefaciens* EHA105MOTUBIROK | Aramir |
| LT-15 | *A. tumefaciens* EHA105MOTGlyKatROK | Aramir |

The material was collected into flasks, and then bathed for 2 min in 70% ethanol. Next, the ethanol was removed, and the seeds were rinsed in sterile water. In a further stage , the seeds were incubated for 20 min. in a 25% solution of the commercial bleach, Clorox with an addition of Tween 20 (at a concentration of 0,01%). Following the required time for sterilisation, the material was washed 4 - 5 times for 2-3 minutes with sterile distilled water, until the Clorox was completely washed out. Sterilised lettuce seeds were placed on dishes with 0,8% agar. The seeds germinated at a temperature of 22 - 24°C, under weak lighting. After 2 to 3 days the cotyls were cut off, and placed in the suspension of A. *tumefaciens* and incubated for 10 min., with gentle mixing. After the inoculation, the cotyls were placed with the ventral side onto LR3A medium. The co-culture was maintained for 2 - 4 days, in the dark at 28°C. Following the end of co-culture the cotyls were transferred onto selective LR3A medium, containing the antibiotics canamycin at 200 mg/l and augmentine at 300 mg/l.
The cotyls on which a callus formed were transferred every 5 days onto fresh LR3A medium containing the antibiotics canamycin at 200 mg/l and augmentine at 300 mg/l. The regenerating plants were excised from the callus, and grafted onto LR2A medium containing the antibiotics canamycin at 200 mg/l and augmentine at 300 mg/l. When the plants reached a size of around 2 - 3 cm, they were transferred into jars containing 1/2SH medium containing the antibiotics canamycin at 200 mg/l and augmentine at 300 mg/l. Rooted plants were transplanted into pots containing sterile sand mixed 1:1 with pearlite and they were adapted to *in vivo* conditions.
Seeds from the primary transformants were sterilised and placed in Petri dishes containing 0,8% agar containing the antibiotics canamycin at 200 mg/l and augmentine at 300 mg/l. The seeds germinated for 1-2 days at a temperature of 28°C in the dark. The saplings developing from seeds were transferred to pots and *in vivo* conditions.

### Plant regeneration

**Table 2. Conditions for the regeneration of plants**

| Plant culture, *in vitro,* culture room | Plant culture, *in vivo,* hothouse |
|---|---|
| - temperature 22 - 24°C | - temperature 18 - 22 °C daytime, 14-18 °C |
| - photoperiod 16/8 | at night |
| - fluorescent light intensity ca. 3000 lux | - photoperiod 14/10 (14h light and 10h darkness) during the fall/winter season and natural during spring and summer. |
| | - fertilizer: nitrogen-phosphate |

### Embodiment 3. Expression and analysis.

### Analysis of transformed bacteria

An initial selection of transformants was made in a medium with the addition of a selection factor, where a resistance factor DNA was introduced (rifampicine 100mg/ml, canamycine 50mg/ml)

The next stage was to analyse the transformants through PCR, using specific oligonucleotide primers.

**Table 3. Oligonucleotide primers used for PCR and sequencing.**

| Gene | Primer | Primer sequence | t° annealing, °C | Product size, bp |
|---|---|---|---|---|
| MotLidC | MOTL5 | | 65 | 240 |
| | MOTL3 | | 65 | |
| MotKatC | MOT5 | | 65 | 546 |
| | MOTK3 | | 65 | |
| MotKatGly | MOTGly5 | | 58 | 1170 |
| | MOTGly3 | | 58 | |
| MotKatUbi | UBIROCK | | 100 | 912 |
| | MOTKATRO | | 100 | |

Confirmation of the presence of transgenes in a vector: a - pMKCROK2, A. *tumefaciens* LBA4404pMKCROK2, b - pMLCROK2, *A. tumefaciens* LBA4404pMLCROK2, c - *A. tumefaciens* EHA105MOTUBIKatROK, d - *A. tumefaciens* EHA105MOTGlyKatROK; is presented in Fig. 15.

Analysis of hybrids of the T0 generation, using PCR.
The resulting transgenic plants were analysed for the presence the hybrid sequence: MotKat - C, MotLid - C, MotKatGly - C and MotKatUbi using PCR. The primers used were: MotL5 and MotL3 specific for the leading domain, Mot5 and MotK3 for the catalytic domain, MOTGly5 and MOTGly3 for the domain containing the glycine bridge, and UBIROCK and MOTKATRO for the ubiquitin containing construct;
Analysis of transformed lettuce plants for the presence of transgenes: a- MotKat -C, b - MotLid - C, c- MotKatGly - C and d- MotKatUbi; is presented in Fig. 16.

The next stage was to produce a genetically homogenous T1 transformant generation. Ten seeds were chosen from 5 F0 transformant lines, and then PCR was used to analyse the plants for the presence of the sequences of the genes introduced. Analysis of the T1 generation of transformed lettuce plants for the presence of transgenes: a- MotKat -C, b - MotLid - C, c- MotKatGly - C and d- MotKatUbi is presented in Fig. 17.

### Immunological analysis of the resultant T1 transformants.

The application of immunological methods allows us to determine the level of expression of a protein.
Hybrid proteins can be detected using anti-C monoclonal antibodies and polyclonal anti-MotKat and anti-MotLid antibodies in a Western blot or a sandwich ELISA.
Proteins were isolated from lyophylisates for Western Blotting. A positive result was obtained, indicating the presence of the proteins in the plants [Figs. 18, 19, 20,21].
Western analysis is not a method which can accurately guage the amount of protein. Furthermore, the protein can undergo fragmentation or aggregation, thus the positive control did not yield one clear band. The protein introduced into the plant is a chimaeric protein, which in addition to the fluke domain also contains a viral protein. This protein has the ability to form structures reminiscent of a viral capsid. Thus, despite the fact that the Western analysis is performed in denaturing conditions, a portion of the domains remains bound, yielding additional signals in the form of an increased number of bands.
A specific ELISA assay was used for the accurate determination of antigen content in plant material.

The Maxisorp (nunc) microwell plate was coated with monoclonal anti-c *(Mab to HBc Ag)* antibody suspended in a carbonate buffer pH=9,6, diluted to 1:5000, and incubated at 37°C for 3 hours. Next, they were rinced three times with phosphate buffer PBS pH=7,4 with 0,05% Tween 20. At a further stage, the plate was blocked with 5% skim milk dissolved in PBS, 390µl per well. After 30 min., the plate was rinsed as described earlier, and coated with the E2 antigen. This stage of the experiment was carried out overnight at 4°C. Next, the plate was rinsed again, and coated with rabbit polyclonal antibodies directed against respectively the leading or catalytic domain of liver fluke cysteine protease suspended in PBS buffer, 100µl/well at a dilution 1:1000 or 1:2000. The plate was incubated at a temperature of 37°C at 100rpm for 1 hour, and then rinsed, and coated with monoclonal anti-mouse IgG (whole molecule) conjugated with alkaline phosphatase (Sigma) suspended in PBS buffer, 100 µl/well at a dilution of 1:20000. The stage was performed at 37°C over 1 hour. Next, the colour-forming reaction was performed by the addition of substrate (INC): 2-amino-2metyl-1,3 propanediol (10-times concentrated) and an aqueous solution of p-nitrophenylphosphate (50-fold concentration) and topped to the desired volume with MQ water. The volume of substrate per well was 100 µl. It was incubated in the dark for 30 min. The readings of the reaction was made at λ=405nm on a BioRad Model 550 plate reader.

T1 generation plants (T1 transformants) were analysed using ELISA for the presence of hybrid proteins in order to select plants which exhibit the highest expression. Only plants with the highest expression of antigen were raised in quantity [Figs. 22a, 22b, 22c, 22d].

The result of the ELISA assay shows expression does not occur in all plants, despite the fact that at the level of DNA they are transgenic. It is therefore justifiable to test the plants immunologically, prior to amassing material for lyophilisation. Thanks to this, it is possible eliminate plants yielding a weak signal, and thus "dilute" the concentration of the antigen in the lyophilisate.

The leaves of plants which gave the strongest signal in the ELISA assay were collected and lyophilised, to condense the material. The resultant material from two experimental variants and containing a leading or catalytic domain fused with protein c of HBV as an antigen were tested via ELISA, to determine the level of antigen for further analysis [Figs. 23a, 23b].

The tests performed show that the selection of plants has resulted in a high level of antigen in the lyophilisate. The specific immunological test has made it possible to determine the level of antigen in the lyophylisates and to plan the experimental immunization of animals. The ELISA assay shows that the level of antigen is around 10 µg in 1 g of lyophilisate containing the leading domain of the gene of cysteine protease fused to protein c of HBV, and 12 µg of antigen per 1 g lyophilisate containing the catalytic domain of the gene of cysteine protease fused to protein c of HBV. Because lyophilisation "concentrates" the antigen around 3-fold, it is admissible that the amount of antigen in 1 g of lyophilisate containing the catalytic domain of the gene of cysteine protease fused to protein c of HBV and the glycine bridge may amount to some 50 µg, and the amount of antigen in 1 g of lyophilisate containing the catalytic domain of the gene of cysteine protease fused to ubiquitin is around 20 µg.

### Embodiment 4. Immunization of mice using transgenic material - lettuce lyophylisate

### 1.Groups of mice

10 week-old females of the Balb/C variety were used in the experiment. The animals were raised in sterile conditions in isolated cages, were given autoclaved water and feed. 6 experimental groups of 8 mice each were set up:
**A-** the group given antigen-containing lyophilisate - hybrid protein composed of the leading domain of the gene of cysteine protease fused to protein c of HBV
**B-** the group given antigen-containing lyophilisate - hybrid protein composed the catalytic domain of the gene of cysteine protease fused to protein c of HBV
**C-** the group given antigen-containing lyophilisate - hybrid protein composed of the catalytic domain of the gene of cysteine protease fused to protein c of HBV protein c of HBV and the glycine bridge
**D-** the group given antigen-containing lyophilisate - hybrid protein composed of the catalytic domain of the gene of cysteine protease fused to ubiquitin
**E-** the group given the lyophilisate of non-transgenic lettuce - the varieties Bipp Burple, Syrena or Aramir
**F-** the group given PBS buffer solution

### 2. Plant material used in the analysis.

The lyophilisate from lettuce leaves of the varieties Bipp Burple, Aramir and Syrena containing the chiameric protein - leading domain of the gene of cysteine protease fused to protein c of HBV (variety Bipp Burple), lyophilisate from lettuce leaves containing the catalytic domain of the gene of cysteine protease fused to protein c of HBV (variety Syrena), lyophilisate from lettuce leaves containing the catalytic domain of the gene of cysteine protease fused to protein c of HBV protein c of HBV and the glycine bridge (variety Aramir) and lyophilisate from lettuce leaves containing the catalytic domain of the gene of cysteine protease fused to ubiquitin (variety Aramir). As a control, lyophylisates of non-transgenic lettuces of the same variety were used, as the transgenic variety. Plant material was suspended in PBS buffer, to a semi-liquid mash consistency (250 µl), whose viscosity facilitated easy administration. The plant material was tolerated well by the animals.

### 3. Antigen administration.

The lyophilisate was administered orally, using a gastric tube. The animals' feed was removed 12 prior to the immunization.

### 4. Schematic of the immunization :

a) The immunised groups **A, B, C** and **D** received lyophylisates of transgenic lettuce. The control group **E** received a lyophilisate of non-transgenic lettuce, and group F received PBS buffer solution.
b) There were two immunisations, with a 30 day interim period.
c) The dose of each immunogen per appropriate group of mice was:
   Antigen dose:
      **1g** of lyophilisate contained:
         MLC -**10 µg** of antigen
         MKC -**12 µg** of antigen
         MKCGly- undetermined, ca. 50 µg
         MKCUbi- undetermined, ca. 20 µg
         Mice were given around 100 ng of antigen (respectively: 10 mg lyophilisate MLC and 8,3 g lyophilisate MKC; and 10 mg MKCGly and MKUbi) suspended in 250 µl PBS. Feed was removed for 12 hours following the immunisation.

### 5. Collection of material from the animals.

150 µl of blood is collected from the periorbital node using a capillary tube, before the experiment, and on days 14, 30 and 45 during the experiment. Faeces samples are collected before immunization, and on days 1, 14 30 and 45 of the experiment.

### 6. Preparation of material for analysis.

### BLOOD:

a) centrifugation immediately following collection in Eppendorf tubes, at 4 °C, 3000 rpm, 20 min
b) the separated serum was kept at -20 °C until time of determination

### FAECES:

a) the material collected was homogenised in PBS at a ratio of 1:5 with a polypropylene pestle
b) it was centrifuged for 10 min, at 4 °C, at 12rpm.
c) the supernatant was separated and stored for further analysis at - 20°C.

### 7. Immunological analysis.

After defrosting, the samples collected were used to carry out assays using a highly sensitive ELISA.
a) A polisorp (Nunc) plate was coated with antigen E2 0.5µg/ml in PBS, at 50µl/well
b) The plate was incubated overnight at 4 °C.
c) It was rinsed three times in PBS with Tween (0,05%), pH 7,4.
d) Blocking was performed with 5% skim milk in PBS for 30 min. at room temperature, 390 µl/well
e) Rinsing as in point c
f) Serum samples were applied in appropriate gradient dilutions, 50 µl/well
g) Incubation took place over 2 hrs. at 37°C, and 60 rpm rotation
h) Rinsing as in point c
i) Incubation with antibodies anti-mouse IgG and IgA conjugated with alkaline phosphatase or peroxidase, 100 µl/well. The antibody dilution was 1:2000., and the incubation time was 1.5 hours at 37°C, and 60 rpm rotation
j) Rinsing as in point c
k) Incubation with substrates for peroxidase or alkaline phosphatase 100µl/well, 1 hour in the dark, at room temperature.
l) The reaction was stopped with 1M sulphuric acid
m) The reaction was read with a BioRad, model 550 plate reader, at λ=405nm.

Analysis of the level of anti-liver fluke antibodies, IgG in the serum and IgA in the faeces, in animals immunised with lyophylisates of transgenic lettuce leaves demonstrate the presence of specific antibodies against the antigen administered - a protein containing a catalytic or leading domain of the cysteine protease of the liver fluke, *Fasciola hepatica.* This would make it a safe assumption that the administration of plant material containing immunogenic, hybrid proteins causes an immune reaction. The method of administration, with food, does not lead to the inactivation, or destruction of immunogenic molecules. which may simplify the future vaccination of animals.

### SEQUENCE LISTING

<110> Institute of Bioorganic Chemistry Polish Academy of Science
   Institute of Parasitology Polish Academy of Science
   Institute of Biotechnology Antibiotics
<120> Chimaeric protein, sequence, construct, plant cell, a method for
   the production of the chimaeric protein and transgenic plant,
   transgenic plant, application of the transgenic plant and
   chimaeric protein, pharmaceutical composition, vaccine and a
   method of induction of immunity
<130> PZ/002/AT/PCT
<140> PCT/PL03/00135
   <141> 2003-12-04
<150> P-357517
   <151> 2002-12-04
<160> 14
<170> Patent In version 3.3
<210> 1
   <211> 740
   <212> DNA
   <213> Artificial sequence
<220>
   <221> source
   <222> (1) .. (740)
   <223> /note="hybrid protein 'core HBV fragment::liver fluke cy steine protease leading domain"'
<400> 1
<210> 2
   <211> 1116
   <212> DNA
   <213> Artificial sequence
<220>
   <221> source
   <222> (1)..(1116)
   <223> /note="hybrid protein 'core HBV fragment ::liver fluke c ysteine protease catalytic domain' "
<400> 2
<210> 3
   <211> 681
   <212> DNA
   <213> Artificial sequence
<220>
   <223> note="hybrid protein 'core HBV fragment ::liver fluke cy steine protease catalytic domain'
<220>
   <221> source
   <222> (1) .. (681)
   <223> /note="liver fluke cysteine protease catalytic domain"
<400> 3
<210> 4
   <211> 686
   <212> DNA
   <213> Artificial sequence
<220>
   <221> source
   <222> (1) .. (686)
   <223> /note="liver fluke cysteine protease catalytic domain introduced into the Bluescript SK(-) vector "
<400> 4
<210> 5
   <211> 699
   <212> DNA
   <213> Artificial sequence
<220>
   <221> source
   <222> (1) .. (699)
   <223> /note="liver fluke cysteine protease catalytic domain introduced into the Bluescript SK(-) vector into places
   arising from digestion with BamHI and HindIII restriction nucleases"
<400> 5
   ggatccatat ggctgtaccc gacaaaattg actggcgtga atctggttat gtgacggagg 60
   tgaaagatca gggaaactgt ggttcctgtt gggcattctc aacaaccggt actatggagg 120
   gacagtatat gaaaaacgaa agaactagta tttcattctc tgagcaacaa ctggtcgatt 180
   gtagcggtcc ttggggaaat aatggttgca gtggtggatt gatggaaaat gcttaccaat 240
   atttgaaaca atttggattg gaaaccgaat cctcttatcc gtacacggct gtggaaggtc 300
   agtgtcgata caataagcag ttaggagttg ccaaagtgac tggctactac actgtgcctt 360
   ctggcagtga ggtagaattg aaaaatctag tcggtgccga aggacctgcc gcggtcgctg 420
   tggatgtgga atctgacttc atgatgtaca ggagtggtat ttatcagagc caaacttgtt 480
   caccgcttcg tgtgaatcat gcagtcttgg ctgtcggtta cggaacacag ggtggtactg 540
   actattggat tgtgaaaaat agttggggat tgtcgtgggg tgagcgcggt tacattcgaa 600
   tggctaggaa tcgaggtaac atgtgtggaa ttgcttcgct ggccagtctc ccgatggtgg 660
   cacgatttcc gggtagcggt agtgatatcg attaagctt 699
<210> 6
   <211> 1127
   <212> DNA
   <213> Artificial sequence
<220>
   <221> source
   <222> (1)..(1127)
   <223> /note="sequence coding the hybrid protein 'core HBV fragment
   ::liver fluke cysteine protease catalytic domain' introduced into
   the ROK2 vector into places arising from digestion with BamHI
   and SacI restriction nucleases"
<400> 6
<210> 7
   <211> 911
   <212> DNA
   <213> Artificial sequence
<220>
   <221> source
   <222> (1)..(911)
   <223> /note="sequence coding the hybrid protein 'ubiquitin::liver fluke
   cysteine protease catalytic domain' introduced into the ROK2
   vector into places arising from digestion with XbaI and BamHI
   restriction nucleases
<400> 7
<210> 8
   <211> 729
   <212> DNA
   <213> Artificial sequence
<220>
   <221> source
   <222> (1) .. (729)
   <223> /note="sequence coding the hybrid protein 'core HBV fragment
   : :liver fluke cysteine protease leading domain' introduced into
   the pIGCmT7 vector into places arising from digestion wi th NdeI
   and HindIII restriction nucleases
<400> 8
<210> 9
   <211> 694
   <212> DNA
   <213> Artificial sequence
<220>
   <223> note="hybrid protein 'core HBV fragment ::liver fluke cysteine
   protease catalytic domain'
<220>
   <221> source
   <222> (1) .. (694)
   <223> /note="sequence coding the liver fluke cysteine protease
   catalytic domain introduced into the pIGCmT7 vector into places
   arising from digestion with NdeI and HindIII restriction nucleases
<400> 9
   catatggctg tacccgacaa aattgactgg cgtgaatctg gttatgtgac ggaggtgaaa 60
   gatcagggaa actgtggttc ctgttgggca ttctcaacaa ccggtactat ggagggacag 120
   tatatgaaaa acgaaagaac tagtatttca ttctctgagc aacaactggt cgattgtagc 180
   ggtccttggg gaaataatgg ttgcagtggt ggattgatgg aaaatgctta ccaatatttg 240
   aaacaatttg gattggaaac cgaatcctct tatccgtaca cggctgtgga aggtcagtgt 300
   cgatacaata agcagttagg agttgccaaa gtgactggct actacactgt gccttctggc 360
   agtgaggtag aattgaaaaa tctagtcggt gccgaaggac ctgccgcggt cgctgtggat 420
   gtggaatctg acttcatgat gtacaggagt ggtatttatc agagccaaac ttgttcaccg 480
   cttcgtgtga atcatgcagt cttggctgtc ggttacggaa cacagggtgg tactgactat 540
   tggattgtga aaaatagttg gggattgtcg tggggtgagc gcggttacat tcgaatggct 600
   aggaatcgag gtaacatgtg tggaattgct tcgctggcca gtctcccgat ggtggcacga 660
   tttccgggta gcggtagtga tatcgattaa gctt 694
<210> 10
   <211> 1198
   <212> DNA
   <213> Artificial sequence
<220>
   <221> source
   <222> (1)..(1198)
   <223> /note="sequence coding the hybrid protein 'core HBV fragment
   ::liver fluke cysteine protease catalytic domain' introduced
   into the pIGCmT7 vector into places arising from digesti on with
   NdeI and HindIII restriction nucleases"
<400> 10
<210> 11
   <211> 1198
   <212> DNA
   <213> Artificial sequence
<220>
   <221> source
   <222> (1) .. (1198)
   <223> /note="sequence coding the hybrid protein 'core HBV frag ment
   ::liver fluke cysteine protease catalytic domain' introduced
   into the Bluescript SK (-) vector into places arising from
   digestion with EcoRI and HindIII restriction nucleases"
<400> 11
<210> 12
   <211> 930
   <212> DNA
   <213> Artificial sequence
<220>
   <221> Source
   <222> (1)..(930)
   <223> /note="sequence coding the hybrid protein 'ubiquitin ::liver
   fluke cysteine protease catalytic domain' introduced into the
   Bluescript SK (-) vector into places arising from digestion with
   EcoRI and XhoI restriction nucleases"
<400> 12
<210> 13
   <211> 740
   <212> DNA
   <213> Artificial sequence
<220>
   <221> source
   <222> (1)..(740)
   <223> /note="sequence coding the hybrid protein 'core HBV frag ment
   .:liver fluke cysteine protease leading domain' introdu ced into
   the ROK2 vector into places arising from digestion with BamHI and
   SacI restriction nucleases"
<400> 13
<210> 14
   <211> 1257
   <212> DNA
   <213> artificial sequence
<220>
   <221> source
   <222> (1) .. (1257)
   <223> /note="sequence coding the hybrid protein 'core HBV fragment
   ::liver fluke cysteine protease catalytic domain' introduced
   into the ROK2 vector into places arising from digestion with
   BamHI and Asp718"
<400> 14

## Claims

1. A nucleic acid molecule coding a chimaeric protein under the control of the constitutive promotor 35S CaMV **characterized in that** the sequence coding for the chimeric protein consists of one of the following sequences:
- a nucleotide sequence coding the amino acid sequence of the catalytic domain of the liver fluke cysteine protease fused to the sequence coding the core protein *HBc,*
- a nucleotide sequence coding the amino acid sequence of the leading domain of the liver fluke cysteine protease fused to the sequence coding the core protein *HBc,*
- a nucleotide sequence coding the amino acid sequence of the catalytic domain of the liver fluke cysteine protease fused to the sequence coding the core protein *HBc,* and glycine residues, or
- a nucleotide sequence coding the amino acid sequence of the catalytic domain of the liver fluke cysteine protease fused to the nucleotide sequence coding ubiquitin.

2. A nucleic acid molecule according to Claim 1, **characterized in that** the sequence for the chimeric protein has been selected from among the sequences presented in Figs. 1 -14.

3. A plant cell which comprises a nucleic acid sequence of claim 1 or 2.

4. A method for the production of a chimaeric protein which encompasses the introduction of a vector into cells of *Agrobacterium tumefaciens* using electroporation, transformation of plants with a strain of bacteria comprising this vector, and the expression of the chimaeric protein, wherein the vector consists of a construct comprising one of the following sequences:
- a nucleotide sequence coding the amino acid sequence of the catalytic domain of the liver fluke cysteine protease fused to the sequence coding the core protein *HBc,*
- a nucleotide sequence coding the amino acid sequence of the leading domain of the liver fluke cysteine protease fused to the sequence coding the core protein *HBc,*
- a nucleotide sequence coding the amino acid sequence of the catalytic domain of the liver fluke cysteine protease fused to the sequence coding the core protein *HBc,* and glycine residues, or
- a nucleotide sequence coding the amino acid sequence of the catalytic domain of the liver fluke cysteine protease fused to the nucleotide sequence coding ubiquitin.

5. A method for the production of a transgenic plant, which encompasses the introduction of a vector into cells of *Agrobacterium tumefaciens* using electroporation, transformation of plants with a strain of bacteria consisting of this vector, and regeneration of the plants, wherein the vector consists of a construct comprising one of the following sequences:
- a nucleotide sequence coding the amino acid sequence of the catalytic domain of the liver fluke cysteine protease fused to the sequence coding the core protein *HBc,*
- a nucleotide sequence coding the amino acid sequence of the leading domain of the liver fluke cysteine protease fused to the sequence coding the core protein *HBc,*
- a nucleotide sequence coding the amino acid sequence of the catalytic domain of the liver fluke cysteine protease fused to the sequence coding the core protein *HBc,* and glycine residues, or
- a nucleotide sequence coding the amino acid sequence of the catalytic domain of the liver fluke cysteine protease fused to the nucleotide sequence coding ubiquitin.

6. A transgenic plant which possesses cells transformed with a construct comprising a nucleotide sequence, **characterized in that** it possesses cells transformed with a construct consisting of one of the following sequences:
- a nucleotide sequence coding the amino acid sequence of the catalytic domain of the liver fluke cysteine protease fused to the sequence coding the core protein *HBc,*
- a nucleotide sequence coding the amino acid sequence of the leading domain of the liver fluke cysteine protease fused to the sequence coding the core protein *HBc,*
- a nucleotide sequence coding the amino acid sequence of the catalytic domain of the liver fluke cysteine protease fused to the sequence coding the core protein *HBc,* and glycine residues, or
- a nucleotide sequence coding the amino acid sequence of the catalytic domain of the liver fluke cysteine protease fused to the nucleotide sequence coding ubiquitin.

7. Use of a transgenic plant transformed with a construct comprising a nucleotide sequence coding the amino acid sequence for a chimeric protein, **characterized in that** the chimaeric protein consists of one of the following sequences:
- the sequence of the catalytic domain of the liver fluke cysteine protease fused to the sequence of the core protein *HBc,*
- the sequence of the leading domain of the liver fluke cysteine protease fused to the sequence of the core protein *HBc,*
- the sequence of the catalytic domain of the liver fluke cysteine protease fused to the sequence of the core protein *HBc* and glycine residues forming the structure of a glycine bridge, or
- the sequence of the catalytic domain of the liver fluke cysteine protease fused to the sequence of ubiquitin.

8. A pharmaceutical composition containing a plant cell comprising a nucleotide sequence coding the amino acid sequence of a chimeric protein or a transgenic plant possessing cells transformed with a nucleotide sequence coding the amino acid sequence of a chimaeric protein or its tissues or their derivative products and possibly a pharmaceutically allowable carrier, **characterized in that** the amino acid sequence of said chimaeric protein consists of one of the following sequences:
- the sequence of the catalytic domain of the liver fluke cysteine protease fused to the sequence of the core protein *HBc,*
- the sequence of the leading domain of the liver fluke cysteine protease fused to the sequence of the core protein *HBc,*
- the sequence of the catalytic domain of the liver fluke cysteine protease fused to the sequence of the core protein *HBc* and glycine residues forming the structure of a glycine bridge, or
- the sequence of the catalytic domain of the liver fluke cysteine protease fused to the sequence of ubiquitin.

9. A pharmaceutical composition according to Claim 8, **characterized in that** it is a lyophilisate.

10. A vaccine against liver fluke containing a plant cell comprising a nucleotide sequence coding the amino acid sequence of a chimeric protein or a transgenic plant possessing cells transformed with a nucleotide sequence coding the amino acid sequence of a chimaeric protein or its tissues or their derivative products and possibly a pharmaceutically allowable carrier, **characterized in that** the amino acid sequence of said chimaeric protein consists of one of the following sequences:
- the sequence of the catalytic domain of the liver fluke cysteine protease fused to the sequence of the core protein *HBc,*
- the sequence of the leading domain of the liver fluke cysteine protease fused to the sequence of the core protein *HBc,*
- the sequence of the catalytic domain of the liver fluke cysteine protease fused to the sequence of the core protein *HBc* and glycine residues forming the structure of a glycine bridge, or
- the sequence of the catalytic domain of the liver fluke cysteine protease fused to the sequence of ubiquitin.

11. A vaccine according to claim 10, **characterized in that** it is a lyophilisate.

12. A vaccine according to claim 13, **characterized in that** it is an oral vaccine.

13. A plant cell of claim 3 or a transgenic plant of claim 6 for use in the induction of immunity against liver fluke infections in mammals.

14. A plant cell according to Claim 13, **characterized in that** the said plant cell is administered orally.

## Patentansprüche

1. Das chimerische Protein unter der Kontrolle des konstitutiven CaMV 35S-Promotors codierende Nukleinsäuremolekül, **dadurch gekennzeichnet, dass** die das chimerische Protein codierende Sequenz aus einer der folgenden Sequenzen besteht:
- der die Aminosäuresequenz der katalytischen Domäne der Zysteinproteinasen aus Fasciola hepatica (großer Leberegel) codierenden Nukleotidsequenz, verbunden mit der das *HBc* Core Protein codierenden Sequenz,
- der die Aminosäuresequenz der Leader Domäne der Zysteinproteinasen aus Fasciola hepatica (großer Leberegel) codierenden Nukleotidsequenz, verbunden mit der das *HBc* Core Protein codierenden Sequenz,
- der die Aminosäuresequenz der katalytischen Domäne der Zysteinproteinasen aus Fasciola hepatica (großer Leberegel) codierenden Nukleotidsequenz, verbunden mit der das *HBc* Core Protein codierenden Sequenz und Glyzinreste, oder
- der die Aminosäuresequenz der katalytischen Domäne der Zysteinproteinasen aus Fasciola hepatica (großer Leberegel) codierenden Nukleotidsequenz, verbunden mit der Ubiquitin codierenden Nukleotidsequenz.

2. Das Nukleinsäuremolekül nach dem Anspruch 1, **dadurch gekennzeichnet, dass** die das chimerische Protein codierende Sequenz von den auf Fig. 1 - Fig. 14. dargestellten Sequenzen ausgewählt wurde.

3. Die Nukleinsäuresequenz enthaltende Pflanzenzelle mit dem Anspruch 1 oder 2.

4. Die Gewinnungsmethode von dem chimerischen Protein, die die Einführung des Vektors in die *Agrobacterium tumefaciens* Zellen mittels der Elektroporationsmethode, die Pflanzentransformation mit Agrobakterien, die den Vektor beinhalten und die Expression des chimerischen Proteins einschließt, wobei der Vektor ein Konstrukt enthält, das eine der folgenden Sequenzen beinhaltet:
- die Aminosäuresequenz der katalytischen Domäne der Zysteinproteinasen aus Fasciola hepatica (großer Leberegel) codierende Nukleotidsequenz, verbunden mit der das *HBc* Core Protein codierenden Sequenz,
- die Aminosäuresequenz der Leader Domäne der Zysteinproteinasen aus Fasciola hepatica (großer Leberegel) codierende Nukleotidsequenz, verbunden mit der das *HBc* Core Protein codierenden Sequenz,
- die Aminosäuresequenz der katalytischen Domäne der Zysteinproteinasen aus Fasciola hepatica (großer Leberegel) codierenden Nukleotidsequenz, verbunden mit der das *HBc* Core Protein codierenden Sequenz und Glyzinreste, oder
- die Aminosäuresequenz der katalytischen Domäne der Zysteinproteinasen aus Fasciola hepatica (großer Leberegel) codierende Nukleotidsequenz, verbunden mit der Ubiquitin codierenden Nukleotidsequenz.

5. Die Gewinnungsmethode von einer transgenischen Pflanze, die die Einführung des Vektors in die *Agrobacterium tumefaciens* Zellen mittels der Elektroporationsmethode, die Pflanzentransformation mit Agrobakterien, die den Vektor beinhalten und die Pflanzenregeneration einschließt, wobei der Vektor ein Konstrukt enthält, das eine der folgenden Sequenzen beinhaltet:
- die Aminosäuresequenz der katalytischen Domäne der Zysteinproteinasen aus Fasciola hepatica (großer Leberegel) codierende Nukleotidsequenz, verbunden mit der das *HBc* Core Protein codierenden Sequenz,
- die Aminosäuresequenz der Leader Domäne der Zysteinproteinasen aus Fasciola hepatica (großer Leberegel) codierende Nukleotidsequenz, verbunden mit der das *HBc* Core Protein codierenden Sequenz,
- die Aminosäuresequenz der katalytischen Domäne der Zysteinproteinasen aus Fasciola hepatica (großer Leberegel) codierende Nukleotidsequenz, verbunden mit der das *HBc* Core Protein codierenden Sequenz und Glyzinreste, oder
- die Aminosäuresequenz der katalytischen Domäne der Zysteinproteinasen aus Fasciola hepatica (großer Leberegel) codierende Nukleotidsequenz, verbunden mit der Ubiquitin codierenden Nukleotidsequenz.

6. Eine transgenische Pflanze, die durch ein Konstrukt transformierte Zellen besitzt, das eine Nukleotidsequenz beinhaltet, **dadurch gekennzeichnet, dass** sie durch ein Konstrukt transformierte Zellen enthält, das eine der folgenden Sequenzen beinhaltet:
- die Aminosäuresequenz der katalytischen Domäne der Zysteinproteinasen aus Fasciola hepatica (großer Leberegel) codierende Nukleotidsequenz, verbunden mit der das *HBc* Core Protein codierenden Sequenz,
- die Aminosäuresequenz der Leader Domäne der Zysteinproteinasen aus Fasciola hepatica (großer Leberegel) codierende Nukleotidsequenz, verbunden mit der das *HBc* Core Protein codierenden Sequenz,
- die Aminosäuresequenz der katalytischen Domäne der Zysteinproteinasen aus Fasciola hepatica (großer Leberegel) codierende Nukleotidsequenz, verbunden mit der das *HBc* Core Protein codierenden Sequenz und Glyzinreste, oder
- die Aminosäuresequenz der katalytischen Domäne der Zysteinproteinasen aus Fasciola hepatica (großer Leberegel) codierende Nukleotidsequenz, verbunden mit der Ubiquitin codierenden Nukleotidsequenz.

7. Der Einsatz einer transgenischen durch ein Konstrukt transformierte Pflanze, das eine Nukleotidsequenz beinhaltet, die eine Aminosäuresequenz des chimerischen Proteins codiert, **dadurch gekennzeichnet, dass** das chimerische Protein aus einer der folgenden Sequenzen besteht:
- der Sequenz der katalytischen Domäne der Zysteinproteinasen aus Fasciola hepatica (großer Leberegel) verbunden mit der *HBc* Core Protein-Sequenz,
- der Sequenz der Leader Domäne der Zysteinproteinasen aus Fasciola hepatica (großer Leberegel) verbunden mit der *HBc* Core Protein-Sequenz,
- der Sequenz der katalytischen Domäne der Zysteinproteinasen aus Fasciola hepatica (großer Leberegel) verbunden mit der *HBc* Core Protein-Sequenz und Glyzinresten, die eine Struktur der Glyzinbrücke bilden oder
- der Sequenz der katalytischen Domäne der Zysteinproteinasen aus Fasciola hepatica (großer Leberegel) verbunden mit der Ubiquitin-Sequenz.

8. Die Pflanzenzelle enthaltende Pharmazeutische Komposition, die eine Aminosäuresequenz des chimerischen Proteins codierende Nukleotidsequenz enthält oder eine transgenische Pflanze, die durch eine Aminosäuresequenz des chimerischen Proteins codierende Nukleotidsequenz transformierte Zellen enthält oder ihr Gewebe oder Produkte seiner Behandlung und eventuell einen pharmazeutisch zulässigen Träger, **dadurch gekennzeichnet, dass** die Aminosäuresequenz des chimerischen Proteins aus einer der folgenden Sequenzen besteht:
- der Sequenz der katalytischen Domäne der Zysteinproteinasen aus Fasciola hepatica (großer Leberegel) verbunden mit der *HBc* Core Protein-Sequenz,
- der Sequenz der Leader Domäne der Zysteinproteinasen aus Fasciola hepatica (großer Leberegel) verbunden mit der *HBc* Core Protein-Sequenz,
- der Sequenz der katalytischen Domäne der Zysteinproteinasen aus Fasciola hepatica (großer Leberegel) verbunden mit der *HBc* Core Protein-Sequenz und Glyzinresten, die eine Struktur der Glyzinbrücke bilden oder
- der Sequenz der katalytischen Domäne der Zysteinproteinasen aus Fasciola hepatica (großer Leberegel) verbunden mit der Ubiquitin-Sequenz.

9. Pharmazeutische Komposition nach dem Anspruch 8, **dadurch gekennzeichnet, dass** sie die Form eines Lyophilisates hat.

10. Der eine Pflanzenzelle enthaltende Impfstoff gegen Fasciola hepatica (großer Leberegel) ,die eine Aminosäuresequenz des chimerischen Proteins codierende Nukleotidsequenz enthält oder eine transgenische Pflanze, die durch eine Aminosäuresequenz des chimerischen Proteins codierende Nukleotidsequenz transformierte Zellen enthält oder ihr Gewebe oder Produkte seiner Behandlung und eventuell einen pharmazeutisch zulässigen Träger, **dadurch gekennzeichnet, dass** die Aminosäuresequenz des chimerischen Proteins aus einer der folgenden Sequenzen besteht:
- der Sequenz der katalytischen Domäne der Zysteinproteinasen aus Fasciola hepatica (großer Leberegel) verbunden mit der *HBc* Core Protein-Sequenz,
- der Sequenz der Leader Domäne der Zysteinproteinasen aus Fasciola hepatica (großer Leberegel) verbunden mit der *HBc* Core Protein-Sequenz,
- der Sequenz der katalytischen Domäne der Zysteinproteinasen aus Fasciola hepatica (großer Leberegel) verbunden mit der *HBc* Core Protein-Sequenz und Glyzinresten, die eine Struktur der Glyzinbrücke bilden, oder
- der Sequenz der katalytischen Domäne der Zysteinproteinasen aus Fasciola hepatica (großer Leberegel) verbunden mit der Ubiquitin-Sequenz.

11. Der Impfstoff nach dem Anspruch 10, **dadurch gekennzeichnet, dass** er die Form eines Lyophilisates hat.

12. Der Impfstoff nach dem Anspruch 10, **dadurch gekennzeichnet, dass** er zum einnehmen ist,

13. Die Pflanzenzelle nach dem Anspruch 3 oder die transgenische Pflanze nach dem Anspruch 6 zum Einsatz für die Induktion von Immunität gegen Fasciola hepatica (großer Leberegel) bei Säugetieren.

14. Pflanzenzelle nach dem Anspruch 13, **dadurch gekennzeichnet, dass** sie über den Verdauungsweg verabreicht wird.

## Revendications

1. La molécule de l'acide nucléique, codant la protéine chimérique sous le contrôle d'un promoteur constitutif 35S CaMV, se **caractérise par** la séquence codant la protéine chimérique qui est composée de l'une des séquences suivantes:
- séquence nucléotide codant la séquence d'acide aminé du domaine catalytique de la cystéine protéinase de la douve du foie, combinée avec la séquence codant la protéine de capside *HBc,*
- séquence nucléotide codant la séquence d'acide aminé du domaine leader de la cystéine protéinase de la douve du foie, combinée avec la séquence codant la protéine de capside *HBc,*
- séquence nucléotide codant la séquence d'acide aminé du domaine catalytique de la cystéine protéinase de la douve du foie, combinée avec la séquence codant la protéine de capside *HBc* et des résidus de glycine ou
- séquence nucléotide codant la séquence d'acide aminé du domaine catalytique de la cystéine protéinase de la douve du foie, combinée avec la séquence nucléotide codant l'ubiquitine.

2. Une molécule de l'acide nucléique selon la revendication no 1, se **caractérise par le fait que** la séquence codant la protéine chimérique a été choisie parmi les séquences présentées sur la figure 1 - figure 14.

3. Une cellule végétale contient une séquence d'acide nucléique de la revendication no 1 ou no 2.

4. La méthode permettant de produire la protéine chimérique consiste en l'introduction du vecteur dans les cellules *Agrobacterium tumefaciens* avec la méthode de l'électroporation, la transformation des plantes par une souche de bactéries comprenant ce vecteur-là et l'expression de la protéine chimérique; le vecteur contient cependant une construction composée de l'une des séquences suivantes:
- séquence nucléotide codant la séquence d'acide aminé du domaine catalytique de la cystéine protéinase de la douve du foie, combinée avec une séquence codant la protéine de capside *HBc,*
- séquence nucléotide codant la séquence d'acide aminé du domaine leader de la cystéine protéinase de la douve du foie, combinée avec une séquence codant la protéine de capside *HBc,*
- séquence nucléotide codant la séquence d'acide aminé du domaine catalytique de la cystéine protéinase de la douve du foie, combinée avec une séquence codant la protéine de capside *HBc* et des résidus de glycine ou
- séquence nucléotide codant la séquence d'acide aminé du domaine catalytique de la cystéine protéinase de la douve du foie, combinée avec une séquence nucléotide codant l'ubiquitine.

5. La méthode permettant de produire une plante transgénique consiste en l'introduction du vecteur dans les cellules *Agrobacterium tumefaciens* avec la méthode de l'électroporation, la transformation de plantes par une souche de bactéries contenant ce vecteur-là et, la régénération des plantes; le vecteur contient cependant une construction composée de l'une des séquences suivantes :
- séquence nucléotide codant la séquence d'acide aminé du domaine catalytique de la cystéine protéinase de la douve du foie, combinée avec une séquence codant la protéine de capside *HBc,*
- séquence nucléotide codant la séquence d'acide aminé du domaine leader de la cystéine protéinase de la douve du foie, combinée avec une séquence codant la protéine de capside *HBc,*
- séquence nucléotide codant la séquence d'acide aminé du domaine catalytique de la cystéine protéinase de la douve du foie, combinée avec une séquence codant la protéine de capside *HBc* et des résidus de glycine ou
- séquence nucléotide codant la séquence d'acide aminé du domaine catalytique de la cystéine protéinase de la douve du foie, combinée avec une séquence nucléotide codant l'ubiquitine.

6. Une plante transgénique, possédant des cellules transformées par une construction qui comprend une séquence nucléotide, se **caractérise par le fait qu'**elle possède des cellules transformées par une construction composée de l'une des séquences suivantes:
- séquence nucléotide codant la séquence d'acide aminé du domaine catalytique de la cystéine protéinase de la douve du foie, combinée avec une séquence codant la protéine de capside *HBc,*
- séquence nucléotide codant la séquence d'acide aminé du domaine leader de la cystéine protéinase de la douve du foie, combinée avec une séquence codant la protéine de capside *HBc*
- séquence nucléotide codant la séquence d'acide aminé du domaine catalytique de la cystéine protéinase de la douve du foie, combinée avec une séquence codant la protéine de capside *HBc* et des résidus de glycine ou
- séquence nucléotide codant la séquence d'acide aminé du domaine catalytique de la cystéine protéinase de la douve du foie, combinée avec une séquence nucléotide codant l'ubiquitine.

7. L'utilisation de la plante transgénique transformée par une construction comprenant une séquence nucléotide codant la séquence d'acide aminé de la protéine chimérique, se **caractérise par** la protéine chimérique qui se compose de l'une des séquences suivantes:
- séquence du domaine catalytique de la cystéine protéinase de la douve du foie, combinée avec une séquence de la protéine de capside *HBc,*
- séquence du domaine leader de la cystéine protéinase de la douve du foie, combinée avec une séquence de la protéine de capside *HBc,*
- séquence du domaine catalytique de la cystéine protéinase de la douve du foie, combinée avec une séquence de la protéine de capside *HBc* et des résidus de glycine créant la structure du pont de glycine ou
- séquence du domaine catalytique de la cystéine protéinase de la douve du foie, combinée avec une séquence de l'ubiquitine.

8. Une composition pharmaceutique contient une cellule végétale constituée d'une séquence nucléotide codant la séquence d'acide aminé de la protéine chimérique ou une plante transgénique possédant des cellules transformées par une séquence nucléotide codant la séquence d'acide aminé de la protéine chimérique ou, ses tissus ou leurs produits dérivés, éventuellement un support admissible du point de vue pharmaceutique, se **caractérise par le fait que** la séquence d'acide aminé de la protéine chimérique est constituée de l'une des séquences suivantes:
- séquence du domaine catalytique de la cystéine protéinase de la douve du foie, combinée avec une séquence de la protéine de capside *HBc,*
- séquence du domaine leader de la cystéine protéinase de la douve du foie, combinée avec une séquence de la protéine de capside *HBc,*
- séquence du domaine catalytique de la cystéine protéinase de la douve du foie, combinée avec une séquence de la protéine de capside *HBc* et des résidus de glycine créant la structure du pont de glycine ou
- séquence du domaine catalytique de la cystéine protéinase de la douve du foie, combinée avec une séquence de l'ubiquitine.

9. Une composition pharmaceutique selon la revendication 8, se **caractérise par** sa forme de lyophilisat.

10. Le vaccin contre la douve du foie comprenant une cellule végétale contenant une séquence nucléotide codant la séquence d'acide aminé de la protéine chimérique ou une plante transgénique possédant des cellules transformées par une séquence nucléotide codant la séquence d'acide aminé de la protéine chimérique ou, ses tissus ou leurs produits dérivés, éventuellement un support admissible du point de vue pharmaceutique, se **caractérise par le fait que** la séquence d'acide aminé de la protéine chimérique est composée de l'une des séquences suivantes:
- séquence du domaine catalytique de la cystéine protéinase de la douve du foie, combinée avec une séquence de la protéine de capside *HBc,*
- séquence du domaine leader de la cystéine protéinase de la douve du foie, combinée avec une séquence de la protéine de capside *HBc,*
- séquence du domaine catalytique de la cystéine protéinase de la douve du foie, combinée avec une séquence de la protéine de capside *HBc* et des résidus de glycine créant la structure du pont de glycine ou
- séquence du domaine catalytique de la cystéine protéinase de la douve du foie, combinée avec une séquence de l'ubiquitine.

11. Le vaccin selon la revendication no 10 se **caractérise par** sa forme de lyophilisat.

12. Le vaccin selon la revendication no 10 se **caractérise par** le fait d'être administré par voie buccale.

13. La cellule végétale selon la revendication no 3 ou la plante transgénique d'après la revendication no 6 peut être utilisée dans l'induction de l'immunité contre la contamination par la douve du foie chez le mammifère.

14. La cellule végétale selon la revendication no 13 se **caractérise par le fait qu'**elle soit administrée par voie digestive.
